# EUROPEAN PATENT APPLICATION

(11) **EP 4 802 994 A1**
(43) Date of publication of application: **09.09.2026**
(21) Application number: 24895688.0
(22) Date of filing: 25.07.2024
(51) Int. Cl.: A61B 5/02

(54) **WEARABLE DEVICE, BLOOD PRESSURE MEASUREMENT METHOD AND RELATED APPARATUS**

(30) Priority: 30.11.2023 CN 202311635159
(71) Applicant: Huawei Technologies Co., Ltd., Shenzhen, Guangdong 518129 (CN)
(72) Inventor: CHEN, Mengji, Shenzhen, Guangdong 518129 (CN); FAN, Wei, Shenzhen, Guangdong 518129 (CN); LIU, Xin, Shenzhen, Guangdong 518129 (CN); ZHANG, Chuan, Shenzhen, Guangdong 518129 (CN); SHI, Jiawei, Shenzhen, Guangdong 518129 (CN); LIU, Yang, Shenzhen, Guangdong 518129 (CN)
(74) Representative: Goddar, Heinz J.
(86) International application number: PCT/CN2024/107600
(87) International publication number: WO 2025/112585

(57) **Abstract**

This application discloses a wearable device, a blood pressure measurement method, and a related apparatus, and pertains to the data processing field. The method includes: obtaining a pressure data set separately collected by at least one sensor array; determining, based on the pressure data set collected by the at least one sensor array, an effective pressure sensing unit from absolute pressure sensing units included in the at least one sensor array; determining, based on the pressure data collected by the effective pressure sensing unit in the at least one sensor array, arterial pressure data respectively corresponding to at least one artery blood vessel; and determining a blood pressure of a user based on the arterial pressure data corresponding to the at least one artery blood vessel. Because the arterial pressure data can represent a pressure actually experienced by a corresponding artery blood vessel in a blood pressure measurement process, accuracy of a blood pressure result determined based on the arterial pressure data corresponding to the artery blood vessel is high.

## Description

This application claims priority to Chinese Patent Application No. 202311635159.6, filed on November 30, 2023 and entitled "WEARABLE DEVICE, BLOOD PRESSURE MEASUREMENT METHOD, AND RELATED APPARATUS", which is incorporated herein by reference in its entirety.

### TECHNICAL FIELD

This application relates to the data processing field, and in particular, to a wearable device, a blood pressure measurement method, and a related apparatus.

### BACKGROUND

A blood pressure is an important indicator for monitoring health and can reflect a health status of a human body. A human blood pressure is a lateral pressure exerted on a blood vessel wall by a pulsating blood flow within a blood vessel, namely, a pressure perpendicular to the blood vessel wall. A peak value of the pressure is a systolic blood pressure, and may alternatively be referred to as a high pressure. A valley value of the pressure is a diastolic blood pressure, and may alternatively be referred to as a low pressure. Currently, the human blood pressure is typically measured by using an upper arm type or wrist type blood pressure monitor. Using the upper arm type blood pressure monitor as an example, a user wears a cuff at a position level with the heart, then inflates and pressurizes an air bladder in the cuff to exceed the systolic blood pressure to block a blood flow in the upper arm, and then gradually deflates and depressurizes the air bladder, thereby collecting pressure data inside the air bladder during a deflation process. The pressure data can represent an actual pressure experienced by an artery blood vessel during a pressurization process, and includes a pulse wave signal of the artery blood vessel (also referred to as a dynamic pressure) and a corresponding external pressurization signal (also referred to as a static pressure). The blood pressure of the user is then determined based on the dynamic pressure and the static pressure.

A width of the air bladder has a decisive influence on precision of blood pressure measurement. For a wrist type blood pressure monitor, the width of the air bladder needs to reach at least 60 millimeters to ensure that collected pressure data accurately represents the actual pressure experienced by the artery blood vessel during the pressurization process, thereby ensuring accuracy of measurement data. However, with advancement and development of technology, increasingly more wearable devices are equipped with a blood pressure measurement function. Due to pursuit of portability and compactness in a wearable device, a width of a wearable strap of the wearable device is usually narrow (typically around 30 millimeters), resulting in a width of an air bladder inside the wearable strap also being narrow (also referred to as a narrow air bladder). When the narrow air bladder is inflated, a cross-section of the narrow air bladder is close to circular, thereby causing compression loss, making collected pressure data higher than the actual pressure experienced by the artery blood vessel, leading to a finally calculated blood pressure that is higher than an actual value. In other words, when the blood pressure is measured with the narrow air bladder, a gas pressure inside the narrow air bladder cannot accurately represent the actual pressure experienced by the artery blood vessel during the pressurization process, thus resulting in an inaccurate final blood pressure measurement result.

### SUMMARY

This application provides a wearable device, a blood pressure measurement method, and a related apparatus, to resolve a problem that a blood pressure measurement result is inaccurate in a related technology. The technical solutions are as follows.

According to a first aspect, a wearable device is provided, where the wearable device includes a wearable strap, an inflatable component, at least one sensor array, and a processor, the inflatable component includes an air pump and an air bladder, the air bladder is on an inner side of the wearable strap, the air bladder is distributed along a length direction of the wearable strap, and the air pump is configured to inflate and pressurize the air bladder and then deflate and depressurize the air bladder in a process in which a user measures a blood pressure; the at least one sensor array is on a side of the air bladder away from the wearable strap, and when the user wears the wearable device, the at least one sensor array corresponds to a position of at least one artery blood vessel of the user; the sensor array includes a plurality of absolute pressure sensing units, where a size of the absolute pressure sensing unit in a first direction is not greater than a diameter of a corresponding artery blood vessel, and the first direction is perpendicular to a flow direction of the corresponding artery blood vessel; and the sensor array is configured to collect, in the process in which the user measures the blood pressure, a pressure experienced by a corresponding artery blood vessel, and the processor is configured to determine the blood pressure of the user based on a pressure data set collected by the at least one sensor array.

Because the sensor array includes a plurality of absolute pressure sensing units, and a pressure measured by the absolute pressure sensing unit is relative to a vacuum pressure, the pressure measured by the absolute pressure sensing units is not affected by a change of an atmospheric pressure and can accurately reflect a real pressure status. Because the at least one sensor array is on the side of the air bladder away from the wearable strap, in this case, when the user wears the wearable device, the sensor array can be in contact with skin of the user, and collect a pressure data at the contact position. The pressure data is relative to the vacuum pressure. Compared with a method for collecting pressure data inside the air bladder, the pressure data collected in this application can accurately represent a pressure actually experienced by a corresponding contact position. Therefore, accuracy of a finally determined blood pressure of the user is ensured.

Optionally, a column direction of the sensor array is the same as a flow direction of the corresponding artery blood vessel, the plurality of absolute pressure sensing units are arranged in a manner of M rows and N columns, a spacing between two adjacent absolute pressure sensing units in a same row is not greater than a diameter of the corresponding artery blood vessel, M is an integer greater than or equal to 1, and N is an integer greater than 1.

For an artery blood vessel corresponding to any sensor array, because a distance between an absolute pressure sensing unit right above the artery blood vessel and the artery blood vessel is the smallest, signal strength of a pulse wave right above the artery blood vessel is the largest, and the signal strength of the pulse wave gradually attenuates from right above the artery blood vessel to two sides. If the size of the absolute pressure sensing unit in the first direction is greater than the diameter of the corresponding artery blood vessel, a difference between pressure data collected by the absolute pressure sensing units in the sensor array is small, and it is difficult to determine, based on the pressure data collected by the absolute pressure sensing units, the arterial pressure data corresponding to the artery blood vessel. However, in this application, the size of the absolute pressure sensing unit in the first direction is not greater than the diameter of the corresponding artery blood vessel. In this way, fidelity of signal details of the pressure data set collected by the sensor array is high, so that the arterial pressure data corresponding to the artery blood vessel is conveniently determined, and the finally determined blood pressure is more accurate.

Optionally, absolute pressure sensing units in two adjacent rows of the plurality of absolute pressure sensing units are staggered.

For an artery blood vessel corresponding to any sensor array, a pulse wave signal strength collected by an absolute pressure sensing unit right above the artery blood vessel is the largest. However, there is usually a specific spacing between two adjacent absolute pressure sensing units in a same row, and a row direction of the sensor array is perpendicular to a flow direction of a corresponding artery blood vessel. Therefore, the artery blood vessel may be in a spacing between two adjacent absolute pressure sensing units in a row of the sensor array, in this case, the two adjacent absolute pressure sensing units cannot be right above the blood vessel, and therefore cannot collect a pulse wave signal with optimal signal strength. However, because absolute pressure sensing units in two adjacent rows of the plurality of absolute pressure sensing units included in the sensor array are staggered, it can be effectively ensured that the sensor array can collect a pulse wave signal with optimal signal strength, thereby ensuring accuracy of the finally determined blood pressure of the user.

Optionally, the at least one sensor array includes a first array and/or a second array, the first array corresponds to a radial artery blood vessel, and the second array corresponds to an ulnar artery blood vessel.

Optionally, a size of the sensor array in the first direction is greater than three times a diameter of the corresponding artery blood vessel.

Absolute pressure sensing units in two adjacent rows of the plurality of absolute pressure sensing units included in the sensor array may be staggered, or a quantity of absolute pressure sensing units included in each row of the sensor array is different and/or a spacing between adjacent absolute pressure sensing units in a same row is different. In this case, the sensor array is not a regular rectangle, to be specific, the sensor array has a plurality of sizes in the first direction. In this case, that the size of the sensor array in the first direction is greater than three times a diameter of the corresponding artery blood vessel may be understood as that a smallest size of a plurality of sizes of the sensor array in the first direction is greater than three times a diameter of the corresponding artery blood vessel.

Optionally, the wearable device further includes a memory, and the memory is configured to store a computer program for performing a blood pressure measurement method provided in a second aspect below. The processor is configured to execute the computer program stored in the memory, to implement the blood pressure measurement method in the second aspect below.

Optionally, the wearable device may further include a communication bus. The communication bus is configured to establish a connection between the processor and the memory.

According to the second aspect, a blood pressure measurement method applied to the wearable device according to the first aspect is provided, where the method includes: obtaining a pressure data set separately collected by the at least one sensor array, where the pressure data set is collected by a plurality of absolute pressure sensing units included in a corresponding sensor array in a process of inflating and pressurizing the air bladder or deflating and depressurizing the air bladder; determining, based on the pressure data set collected by the at least one sensor array, an effective pressure sensing unit from absolute pressure sensing units included in the at least one sensor array, where pressure data collected by the effective pressure sensing unit is able to effectively represent a pulsation status of an artery blood vessel of the user; determining, based on the pressure data collected by the effective pressure sensing unit in the at least one sensor array, arterial pressure data respectively corresponding to the at least one artery blood vessel, where the arterial pressure data represents a pressure actually experienced by a corresponding artery blood vessel in a blood pressure measurement process; and determining a blood pressure of the user based on the arterial pressure data corresponding to the at least one artery blood vessel.

In this application, considering that a size of an artery blood vessel is smaller relative to a size of a sensor array, in this application, an absolute pressure sensing unit (also referred to as an effective pressure sensing unit) that can effectively represent an artery blood vessel pulsation status of a user is determined from a plurality of absolute pressure sensing units included in the sensor array, and then arterial pressure data respectively corresponding to the artery blood vessel is determined based on pressure data collected by the effective pressure sensing unit. In this way, accuracy of finally determined arterial pressure data is ensured. In addition, because the arterial pressure data can represent a pressure actually experienced by a corresponding artery blood vessel in a blood pressure measurement process, accuracy of a blood pressure result determined based on the arterial pressure data corresponding to the artery blood vessel is high. In addition, when arterial pressure data corresponding to at least two artery blood vessels is determined, in this application, the blood pressure of the user can be further determined based on the arterial pressure data corresponding to the at least two artery blood vessels, thereby further improving accuracy of blood pressure measurement.

In a process in which the user measures the blood pressure, the air bladder in the wearable device can perform inflation and pressurization at a target rate and then deflation and depressurization, or perform inflation and pressurization and then deflation and depressurization at a target rate, or perform inflation and pressurization at a target rate and then deflation and depressurization at the target rate. In other words, during two processes of inflation and pressurization and deflation and depressurization of the air bladder in the wearable device, there is at least one process in which the pressure changes at the target rate, and the at least one sensor array can collect a pressure data set during the process in which the pressure of the air bladder changes at the target rate.

The target rate is set in advance. An upper limit of a value range of the target rate is related to a sampling frequency of the sensor array and a heart rate of the user. A higher sampling frequency of the sensor array and/or a higher heart rate of the user indicate a higher upper limit of the value range of the target rate. In other words, the sampling frequency of the sensor array and the heart rate of the user are in direct proportion to the upper limit of the value range of the target rate.

Optionally, the pressure data set includes a plurality of groups of pressure data collected by a plurality of absolute pressure sensing units included in a corresponding sensor array, and each group of pressure data includes pressure data at a plurality of moments.

In other words, for any sensor array, a pressure data set collected by the sensor array includes a plurality of groups of pressure data, the plurality of groups of pressure data are in a one-to-one correspondence with a plurality of absolute pressure sensing units included in the sensor array, and each group of pressure data is pressure data separately collected by a corresponding absolute pressure sensing unit at a plurality of moments.

Optionally, the wearable device determines, based on a first pressure data set, a plurality of groups of candidate pressure sensing units from absolute pressure sensing units included in a first sensor array, and determines an effective pressure sensing unit in the first sensor array from the plurality of groups of candidate pressure sensing units.

The first pressure data set is a pressure data set collected by the first sensor array, the first sensor array is any one of the at least one sensor array included in the wearable device, the plurality of groups of candidate pressure sensing units are in a one-to-one correspondence with a plurality of first moments, positions of a same group of candidate pressure sensing units are consecutive, and a difference between pressure data collected by the same group of candidate pressure sensing units at a corresponding first moment falls within a pressure fluctuation range, where the first moment is one of the plurality of moments, and the first artery blood vessel is an artery blood vessel corresponding to the first sensor array.

Optionally, the wearable device stores coordinates of each absolute pressure sensing unit in the first sensor array. In this case, that the positions of the same group of candidate pressure sensing units are consecutive means that horizontal coordinates and/or vertical coordinates of the same group of candidate pressure sensing units are consecutive. In addition, if a difference between pressure data collected by the same group of candidate pressure sensing units at a same moment falls within a pressure fluctuation range, it indicates that the difference between the pressure data collected by the same group of candidate pressure sensing units is small.

In actual application, a pressure at a position at which the air bladder contacts the skin is far greater than a pressure at a position at which the air bladder does not contact the skin, and a difference between pressure data at the position at which the air bladder contacts the skin is small. Therefore, if positions of the same group of candidate pressure sensing units are consecutive and a difference between collected pressure data is small, it indicates that the group of candidate pressure sensing units may be at a position at which the air bladder contacts the skin. In this case, the pressure data collected by the group of candidate pressure sensing units is effective. In this way, accuracy and reliability of subsequently determined blood pressure of the user can be ensured.

Optionally, an implementation process of determining the effective pressure sensing unit in the first sensor array from the plurality of groups of candidate pressure sensing units includes: determining at least one first sensing unit from a plurality of groups of candidate pressure sensing units, determining, based on the first pressure data set, at least one second sensing unit from absolute pressure sensing units included in the first sensor array, where positions of the at least one second sensing unit are consecutive, and using an intersection set of the at least one first sensing unit and the at least one second sensing unit as the effective pressure sensing unit in the first sensor array.

Based on a dynamic pressure collected by each absolute pressure sensing unit in the first sensor array, a maximum peak-to-peak value corresponding to each absolute pressure sensing unit in the first sensor array is determined to obtain a plurality of maximum peak-to-peak values; at least one second candidate sensing unit is determined based on the plurality of maximum peak-to-peak values, where the at least one second candidate sensing unit is consecutive in position, and a difference between corresponding maximum peak-to-peak values is within a peak-to-peak fluctuation range; and at least one second sensing unit is determined based on the at least one second candidate sensing unit.

For an artery blood vessel corresponding to any sensor array, signal strength of a pulse wave right above the artery blood vessel is the largest, and the signal strength of the pulse wave gradually attenuates from right above the artery blood vessel to two sides. Therefore, if positions of at least one second candidate sensing unit are consecutive and a difference between corresponding maximum peak-to-peak values is small, it indicates that the at least one second candidate sensing unit may be around the artery blood vessel. In this case, only pressure data collected by the at least one second candidate sensing unit is effective, or in other words, data collected by the group of candidate pressure sensing units is pressure data that can represent artery blood vessel pulsation. In this way, accuracy and reliability of subsequently determined blood pressure of the user can be ensured.

In addition, because the first sensing unit is at the position at which the air bladder contacts the skin, the second sensing unit is around the artery blood vessel, and the effective pressure sensing unit in the first sensor array is an intersection set of the at least one first sensing unit and the at least one second sensing unit, the effective pressure sensing unit is an absolute pressure sensing unit at the position at which the air bladder contacts the skin and around the artery blood vessel. In this case, arterial pressure data determined in a subsequent step based on the pressure data collected by the effective pressure sensing unit is accurate and effective, thereby further ensuring accuracy and reliability of the subsequently determined blood pressure of the user.

Optionally, the arterial pressure data corresponding to the second artery blood vessel is determined based on the pressure data collected by the effective pressure sensing unit in the second sensor array, where the second sensor array is any one of the at least one sensor array, and the second artery blood vessel is an artery blood vessel corresponding to the second sensor array.

There are a plurality of implementations of determining, based on the pressure data collected by the effective pressure sensing unit in the second sensor array, the arterial pressure data corresponding to the second artery blood vessel. The following describes two implementations.

**In a first implementation,** the pressure data is pressure data separately collected by the effective pressure sensing unit at a plurality of moments, and the arterial pressure data is arterial pressure data respectively corresponding to the plurality of moments. In this case, for any one of the plurality of moments, a mean value or a maximum value of pressure data collected by the effective pressure sensing unit at the moment is used as arterial pressure data corresponding to the moment, and the arterial pressure data corresponding to the plurality of moments can be obtained in a same manner.

**In a second implementation,** pressure data collected by an effective pressure sensing unit in the second sensor array is determined as the arterial pressure data corresponding to the second artery blood vessel.

Optionally, a maximum peak-to-peak value corresponding to each effective pressure sensing unit is determined based on pressure data collected by the effective pressure sensing unit in the second sensor array, to obtain at least one maximum peak-to-peak value, and pressure data collected by an effective pressure sensing unit corresponding to a largest maximum peak-to-peak value in the at least one maximum peak-to-peak value is used as the arterial pressure data corresponding to the second artery blood vessel.

In actual application, before determining, based on the pressure data collected by the effective pressure sensing unit in the at least one sensor array, the arterial pressure data respectively corresponding to the at least one artery blood vessel, the wearable device may further determine a tissue attenuation coefficient corresponding to at least one effective pressure sensing unit in a target sensor array, where the tissue attenuation coefficient indicates an attenuation status of a pulse wave of a target artery blood vessel caused by a human tissue, the target artery blood vessel is an artery blood vessel corresponding to the target sensor array, and the target sensor array is any sensor array in the at least one sensor array; and correct, based on the tissue attenuation coefficient corresponding to the at least one effective pressure sensing unit, pressure data collected by the at least one effective pressure sensing unit.

In other words, in this application, considering attenuation of the pulse wave of the target artery blood vessel by the human tissue, the pressure data collected by the effective pressure sensing unit is corrected by calculating the tissue attenuation coefficient, so that the corrected pressure data collected by the effective pressure sensing unit can accurately reflect a pulse of the artery blood vessel, thereby ensuring accuracy and reliability of finally determined blood pressure of the user.

A distance between the at least one effective pressure sensing unit and the target artery blood vessel is determined, and a unit attenuation coefficient is determined, where the unit attenuation coefficient is an attenuation status of the pulse wave of the target artery blood vessel caused by a human tissue with a unit thickness; and the tissue attenuation coefficient respectively corresponding to the at least one effective pressure sensing unit is determined based on the unit attenuation coefficient and the distance between the at least one effective pressure sensing unit and the target artery blood vessel.

Optionally, a distance between a first effective pressure sensing unit and the target artery blood vessel is determined, and a unit attenuation coefficient is determined. The first effective pressure sensing unit is any effective pressure sensing unit in the at least one effective pressure sensing unit, and a value obtained by multiplying the unit attenuation coefficient by the distance between the first effective pressure sensing unit and the target artery blood vessel is used as a tissue attenuation coefficient corresponding to the first effective pressure sensing unit. Each first effective pressure sensing unit in the at least one first effective pressure sensing unit is processed in a same manner, so that the tissue attenuation coefficient respectively corresponding to the at least one effective pressure sensing unit can be determined.

A primary pressure sensing unit and a secondary pressure sensing unit in the effective sensing units of the target sensor array are determined based on pressure data collected by the effective pressure sensing units in the target sensor array, where the primary pressure sensing unit is an absolute pressure sensing unit with highest signal strength in the effective sensing units of the target sensor array, and signal strength of the secondary pressure sensing unit is less than signal strength of the primary pressure sensing unit, and based on the primary pressure sensing unit and the secondary pressure sensing unit in the effective sensing units, a distance between the first effective pressure sensing unit and the target artery blood vessel is determined, and the unit attenuation coefficient is determined.

A maximum peak-to-peak value corresponding to each effective pressure sensing unit is determined based on pressure data collected by the effective pressure sensing unit in the target sensor array, to obtain at least one maximum peak-to-peak value, and an effective pressure sensing unit corresponding to a largest maximum peak-to-peak value in the at least one maximum peak-to-peak value is used as the primary pressure sensing unit. An effective pressure sensing unit corresponding to a maximum peak-to-peak value, in the at least one maximum peak-to-peak value, that is not equal to the largest maximum peak-to-peak value and for which a difference is greater than a maximum peak-to-peak value difference threshold, is used as the secondary pressure sensing unit.

If the first effective pressure sensing unit is the primary pressure sensing unit, an implementation of determining the distance between the first effective pressure sensing unit and the target artery blood vessel and determining the unit attenuation coefficient is different from that of determining the first effective pressure sensing unit as the secondary pressure sensing unit, which are separately described below.

**If the first effective pressure sensing unit is the primary pressure sensing unit,** the distance between the first effective pressure sensing unit and the target artery blood vessel and a distance between a target secondary pressure sensing unit and the target artery blood vessel are determined based on the pressure data collected by the first effective pressure sensing unit, pressure data collected by the target secondary pressure sensing unit, and a distance between the first effective pressure sensing unit and the target secondary pressure sensing unit in the first direction, where the target secondary pressure sensing unit is any secondary pressure sensing unit in the target sensor array, and the unit attenuation coefficient is determined based on the pressure data collected by the first effective pressure sensing unit, the pressure data collected by the target secondary pressure sensing unit, the distance between the first effective pressure sensing unit and the target artery blood vessel, and the distance between the target secondary pressure sensing unit and the target artery blood vessel.

**If the first effective pressure sensing unit is the secondary pressure sensing unit,** the distance between the first effective pressure sensing unit and the target artery blood vessel and a distance between the primary pressure sensing unit and the target artery blood vessel are determined based on the pressure data collected by the first effective pressure sensing unit, pressure data collected by the primary pressure sensing unit, and a distance between the first effective pressure sensing unit and the primary pressure sensing unit in the first direction; and the unit attenuation coefficient is determined based on the pressure data collected by the first effective pressure sensing unit, the pressure data collected by the primary pressure sensing unit, the distance between the first effective pressure sensing unit and the target artery blood vessel, and the distance between the primary pressure sensing unit and the target artery blood vessel.

For any effective pressure sensing unit of the at least one effective pressure sensing unit, a tissue attenuation coefficient corresponding to the effective pressure sensing unit is subtracted from pressure data of the effective pressure sensing unit separately in a plurality of absolute pressures to obtain a plurality of corrected static pressures, the tissue attenuation coefficient corresponding to the effective pressure sensing unit is added to the pressure data of the effective pressure sensing unit separately in a plurality of dynamic pressures to obtain a plurality of corrected dynamic pressures, and the plurality of corrected static pressures and the plurality of corrected dynamic pressures are used as first pressure data of the effective pressure sensing unit, so as to implement correction of the pressure data collected by the effective pressure sensing unit. Each effective pressure sensing unit of the at least one effective pressure sensing unit is processed in a same manner, so as to implement correction of the pressure data collected by each effective pressure sensing unit of the at least one effective pressure sensing unit.

For a pressure (that is, a static pressure) applied by the air bladder, the pressure applied by the air bladder first reaches the absolute pressure sensing unit, and then reaches the target artery blood vessel after being attenuated by the human tissue. A static pressure experienced by the target artery blood vessel is less than a static pressure collected by the absolute pressure sensing unit. Therefore, the tissue attenuation coefficient corresponding to the effective pressure sensing unit can be separately subtracted from the plurality of static pressures, so that the static pressures can be corrected.

For a pulse wave generated by the target artery blood vessel, the pulse wave can reach the absolute pressure sensing unit only after being emitted from the target artery blood vessel and attenuated by the human tissue. A dynamic pressure generated by the target artery blood vessel is greater than a dynamic pressure collected by the absolute pressure sensing unit. Therefore, the tissue attenuation coefficient corresponding to the effective pressure sensing unit is separately added to the plurality of dynamic pressures, so that the dynamic pressures can be corrected.

When a quantity of the at least one artery blood vessel is one, the wearable device may directly determine the blood pressure of the user based on the arterial pressure data corresponding to the artery blood vessel and according to a related algorithm.

When the quantity of the at least one artery blood vessel is at least two, that is, the at least one artery blood vessel includes at least two artery blood vessels, there are a plurality of implementations of determining the blood pressure of the user based on arterial pressure data corresponding to the at least two artery blood vessels. The following describes two implementations.

**In a first implementation,** the arterial pressure data includes static pressures respectively corresponding to a plurality of moments and a dynamic pressure corresponding to each static pressure, the static pressure represents a pressure applied by the air bladder, the dynamic pressure represents a pulsation status of a corresponding artery blood vessel under compression of the static pressure, and the at least two artery blood vessels are respectively corresponding to different weights. In this case, dynamic pressures corresponding to a same moment in the dynamic pressures of the arterial pressure data respectively corresponding to the at least two artery blood vessels are separately multiplied by respective corresponding weights, and then are added to obtain a plurality of superposed dynamic pressures, static pressures corresponding to a same moment in the dynamic pressures of the arterial pressure data respectively corresponding to the at least two artery blood vessels are separately multiplied by respective corresponding weights, and then are added to obtain a plurality of superposed static pressures, the plurality of superposed dynamic pressures and the plurality of superposed static pressures are used as the superposed arterial pressure data, and the blood pressure of the user is determined based on the superposed arterial pressure data and according to a related algorithm.

**In a second implementation,** the arterial pressure data includes a plurality of static pressures and a dynamic pressure corresponding to each static pressure, the static pressure represents a pressure applied by the air bladder, and the dynamic pressure represents a pulsation status of a corresponding artery blood vessel under compression of the static pressure. In this case, the wearable device may superpose, based on the static pressure in the arterial pressure data respectively corresponding to the at least two artery blood vessels, the dynamic pressure in the arterial pressure data respectively corresponding to the at least two artery blood vessels, to obtain superposed arterial pressure data, and determine the blood pressure of the user based on the superposed arterial pressure data and according to a related algorithm.

When the at least two artery blood vessels are an ulnar artery and a radial artery, due to different depths of the ulnar artery and the radial artery in the human tissue, irregular bones of the wrist of the human body, and impact of a wearing manner of the user, a pulse wave signal in the air bladder obtained by superimposing pulse pulsation of the ulnar artery and the radial artery in time domain cannot accurately represent a pulsation status of the artery blood vessel of the user in a pressurization process, and has a large error. Consequently, accuracy of a blood pressure result is poor. In this application, the arterial pressure data respectively corresponding to the at least two artery blood vessels can be superposed in a static pressure dimension, thereby fundamentally avoiding a problem that a pulse wave signal error is large due to simple superposition in time domain, and further improving accuracy of blood pressure measurement.

According to a third aspect, a blood pressure measurement apparatus is provided, and is included in the wearable device according to the first aspect. The blood pressure measurement apparatus has a function of implementing behavior of the blood pressure measurement method in the second aspect. The blood pressure measurement apparatus includes at least one module, and the at least one module is configured to implement the blood pressure measurement method provided in the second aspect.

According to a fourth aspect, a computer-readable storage medium is provided. The storage medium stores instructions, and when the instructions are run on a computer, the computer is enabled to perform steps of the blood pressure measurement method according to the second aspect.

According to a fifth aspect, a computer program product including instructions is provided. When the instructions are run on a computer, the computer is enabled to perform steps of the blood pressure measurement method according to the first aspect. In other words, a computer program is provided. When the computer program is run on a computer, the computer is enabled to perform steps of the blood pressure measurement method according to the second aspect.

Technical effects achieved in the third aspect, the fourth aspect, and the fifth aspect are similar to technical effects achieved by corresponding technical means in the first aspect and the second aspect. Details are not described herein again.

### BRIEF DESCRIPTION OF DRAWINGS

FIG. 1 is a diagram of pressure distribution according to an embodiment of this application;
FIG. 2 is a diagram of a wrist type blood pressure monitor according to an embodiment of this application;
FIG. 3 is a diagram of a wearable device according to an embodiment of this application;
FIG. 4 is a diagram of a sensor array according to an embodiment of this application;
FIG. 5 is a diagram of another sensor array according to an embodiment of this application;
FIG. 6 is a diagram of a sensor array a according to an embodiment of this application;
FIG. 7 is a diagram of another sensor array a according to an embodiment of this application;
FIG. 8 is a diagram of an absolute pressure sensing unit according to an embodiment of this application;
FIG. 9 is a diagram of another absolute pressure sensing unit according to an embodiment of this application;
FIG. 10 is a diagram of a sensor array arrangement manner according to an embodiment of this application;
FIG. 11 is a diagram of a spacing between two adjacent absolute pressure sensing units according to an embodiment of this application;
FIG. 12 is a diagram of another sensor array arrangement manner according to an embodiment of this application;
FIG. 13 is a diagram of a flow direction of an artery blood vessel according to an embodiment of this application;
FIG. 14 is a diagram of a first array and a second array according to an embodiment of this application;
FIG. 15 is a diagram of a structure of another wearable device according to an embodiment of this application;
FIG. 16 is a flowchart of a blood pressure measurement method according to an embodiment of this application;
FIG. 17 is a diagram of first pressure data according to an embodiment of this application;
FIG. 18 is a diagram of a pulse wave signal of an artery blood vessel according to an embodiment of this application;
FIG. 19 is a diagram of a primary pressure sensing unit and a secondary pressure sensing unit according to an embodiment of this application;
FIG. 20 is a diagram of superposed arterial pressure data according to an embodiment of this application; and
FIG. 21 is a diagram of a structure of a blood pressure measurement apparatus according to an embodiment of this application.

### DESCRIPTION OF EMBODIMENTS

To make objectives, technical solutions, and advantages of embodiments of this application clearer, the following further describes implementations of this application in detail with reference to the accompanying drawings.

For ease of understanding, before the blood pressure measurement method provided in embodiments of this application is described in detail, an application scenario in embodiments of this application is first described.

A human blood pressure is a lateral pressure exerted on a blood vessel wall by a pulsating blood flow within a blood vessel, namely, a pressure perpendicular to the blood vessel wall. A peak value of the pressure is a systolic blood pressure, and may alternatively be referred to as a high pressure. A valley value of the pressure is a diastolic blood pressure, and may alternatively be referred to as a low pressure. The blood pressure is an important indicator for monitoring health, and can be used to evaluate a health status of a human body and a change of a condition of a critically ill patient. For example, a level of the blood pressure can reflect whether a plurality of indicators such as a cardiac function, a blood flow, a blood volume, and a vascular vasomotor function are normal. When the blood pressure shows an abnormal increase or decrease, it indicates that the above indicators may be abnormal. A sudden decrease in the blood pressure may be caused by an insufficient blood volume, abnormal vasodilation, or a severely impaired cardiac function. In addition, if the blood pressure remains at a high or low level for a long time, it may also cause considerable damage to blood vessels and a plurality of organs throughout the body. Therefore, regular blood pressure measurement is very important, as it can enable early detection of a chronic disease such as hypertension, allowing timely intervention to prevent the condition from worsening. In addition, it can also facilitate timely detection of hypotension to prevent accidents. For a patient with hypertension, regular blood pressure measurement also helps a doctor adjust a treatment plan based on a measurement result, thereby achieving better therapeutic effects.

Currently, the human blood pressure is typically measured by using an upper arm type or wrist type blood pressure monitor. Using the upper arm type blood pressure monitor as an example, a user wears a cuff at a position level with the heart, then inflates and pressurizes an air bladder in the cuff to exceed the systolic blood pressure to block a blood flow in the upper arm, and then gradually deflates and depressurizes the air bladder, thereby collecting pressure data inside the air bladder during a deflation process. The pressure data can represent an actual pressure experienced by an artery blood vessel during a pressurization process, and includes a pulse wave signal of the artery (also referred to as a dynamic pressure) and a corresponding external pressurization signal (also referred to as a static pressure). The blood pressure of the user is then determined based on the dynamic pressure and the static pressure.

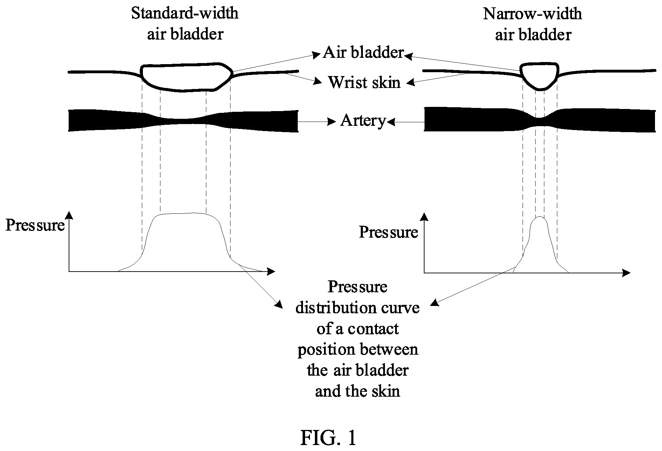

A width of the air bladder has a decisive influence on precision of blood pressure measurement. For a wrist type blood pressure monitor, the width of the air bladder needs to reach at least a standard width (60 millimeters) to ensure that collected pressure data accurately represents the actual pressure experienced by the artery blood vessel during the pressurization process, thereby ensuring accuracy of measurement data. However, with advancement and development of technology, increasingly more wearable devices are equipped with a blood pressure measurement function. Due to pursuit of portability and compactness in a wearable device, a width of a wearable strap of the wearable device is usually narrow (typically around 30 millimeters), resulting in a width of an air bladder inside the wearable strap also being narrow (also referred to as a narrow air bladder/narrow-width air bladder). When the narrow air bladder is inflated, a cross-section of the narrow air bladder is close to circular, thereby causing compression loss, making collected pressure data higher than the actual pressure experienced by the artery blood vessel. In addition, refer to FIG. 1. It is not difficult to see from FIG. 1 that pressure distribution of a position at which the narrow air bladder contacts the skin is not uniform relative to a standard-width air bladder. In this case, a pressure at the position at which the narrow air bladder contacts the skin is different from a gas pressure inside the air bladder, and consequently, a finally calculated blood pressure is higher than an actual value. In other words, when the blood pressure is measured with the narrow air bladder, a gas pressure inside the narrow air bladder cannot accurately represent the actual pressure experienced by the artery blood vessel during the pressurization process, thus resulting in an inaccurate final blood pressure measurement result.

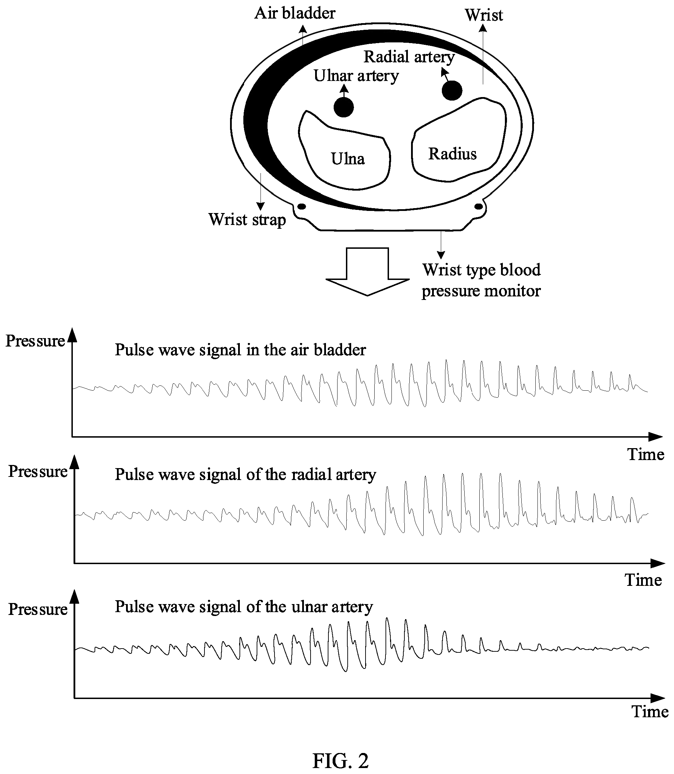

In addition, refer to FIG. 2. For a wrist type blood pressure monitor, a pulse wave signal included in pressure data inside the air bladder collected by the wrist type blood pressure monitor is actually a result of pulsation of an ulnar artery and a radial artery acting together on the air bladder. For ease of description, subsequently, the pulse wave signal included in the pressure data inside the air bladder is referred to as a pulse wave signal in the air bladder. In other words, refer to FIG. 2. The pulse wave signal in the air bladder is actually a superposition of a pulse wave signal of the ulnar artery and a pulse wave signal of the radial artery in time domain. In an ideal case, waveforms of the pulse wave signal of the ulnar artery and the pulse wave signal of the radial artery need to be close, and mean arterial pressures (mean arterial pressure, MAP) respectively corresponding to the ulnar artery and the radial artery also need to be close to a mean arterial pressure of the user. However, due to different depths of the ulnar artery and the radial artery in the human tissue, irregular bones of the wrist of the human body, impact of a wearing manner of the user, and the like, the narrow air bladder exerts different pressures on the ulnar artery and the radial artery at a same moment. In this case, a pulse wave signal in the air bladder obtained by superimposing pulse pulsation of the ulnar artery and the radial artery in time domain cannot accurately represent a pulsation status of the artery blood vessel of the user in a pressurization process, and has a large error. This problem further causes poor accuracy of a blood pressure result.

Based on the foregoing problem, an embodiment of this application provides a wearable device. The wearable device includes a wearable strap, an inflatable component, at least one sensor array, and a processor. The inflatable component includes an air pump and an air bladder. Because the sensor array includes a plurality of absolute pressure sensing units, and a pressure measured by the absolute pressure sensing unit is relative to a vacuum pressure, the pressure measured by the absolute pressure sensing units is not affected by a change of an atmospheric pressure and can accurately reflect a real pressure status. Because the at least one sensor array is on the side of the air bladder away from the wearable strap, in this case, when the user wears the wearable device, the sensor array can be in contact with skin of the user, and collect a pressure data at the contact position. The pressure data is relative to the vacuum pressure. Compared with a method for collecting pressure data inside the air bladder, the pressure data collected in this embodiment of this application can accurately represent a pressure actually experienced by a corresponding contact position. Therefore, accuracy of a finally determined blood pressure of the user is ensured. In addition, in this embodiment of this application, considering that a size of an artery blood vessel is smaller relative to a size of a sensor array, in this embodiment of this application, an absolute pressure sensing unit (also referred to as an effective pressure sensing unit) that can effectively represent an artery blood vessel pulsation status of a user is determined from a plurality of absolute pressure sensing units included in the sensor array, and then arterial pressure data respectively corresponding to the artery blood vessel is determined based on pressure data collected by the effective pressure sensing unit. In this way, accuracy of finally determined arterial pressure data is ensured. In addition, because the arterial pressure data can represent a pressure actually experienced by a corresponding artery blood vessel in a blood pressure measurement process, accuracy of a blood pressure result determined based on the arterial pressure data corresponding to the artery blood vessel is high. In addition, when arterial pressure data corresponding to at least two artery blood vessels is determined, in this embodiment of this application, the blood pressure of the user can be further determined based on the arterial pressure data corresponding to the at least two artery blood vessels, thereby further improving accuracy of blood pressure measurement.

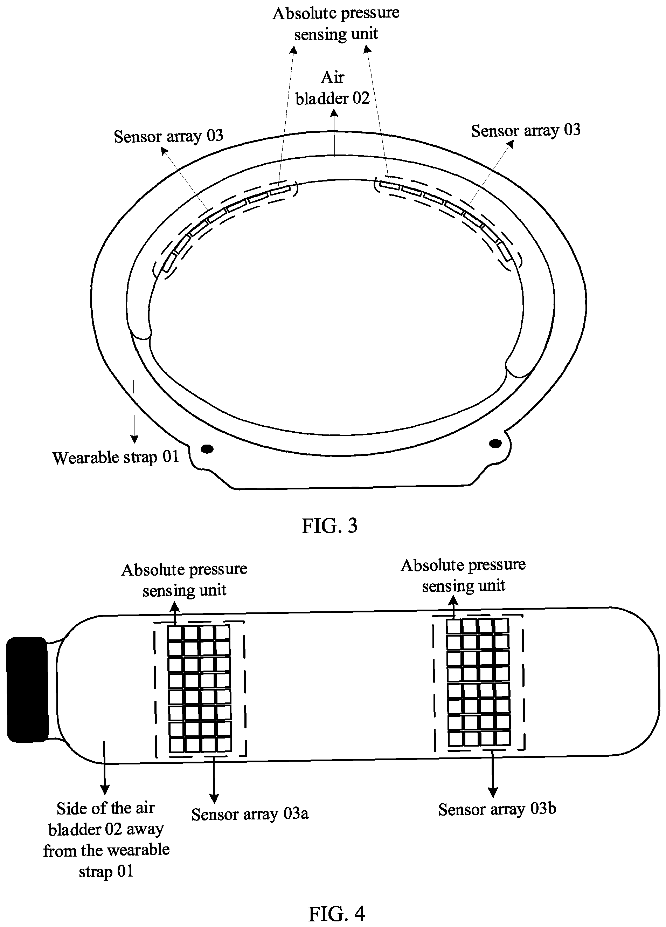

FIG. 3 is a diagram of a wearable device according to an embodiment of this application. The wearable device includes a wearable strap 01, an air pump (not shown in FIG. 3) and an air bladder 02 that are included in an inflatable component, at least one sensor array 03 (in FIG. 3, two sensor arrays schematically represent the at least one sensor array), and a processor (not shown in FIG. 3).

The air bladder 02 is on an inner side of the wearable strap 01, and the air bladder 02 is distributed along a length direction of the wearable strap 01. The air pump is configured to inflate and pressurize the air bladder 02 and then deflate and depress the air bladder 02 in a process in which a user measures a blood pressure. The at least one sensor array 03 is on a side of the air bladder 02 away from the wearable strap 01. When the user wears the wearable device, the at least one sensor array corresponds to a position of at least one artery blood vessel of the user.

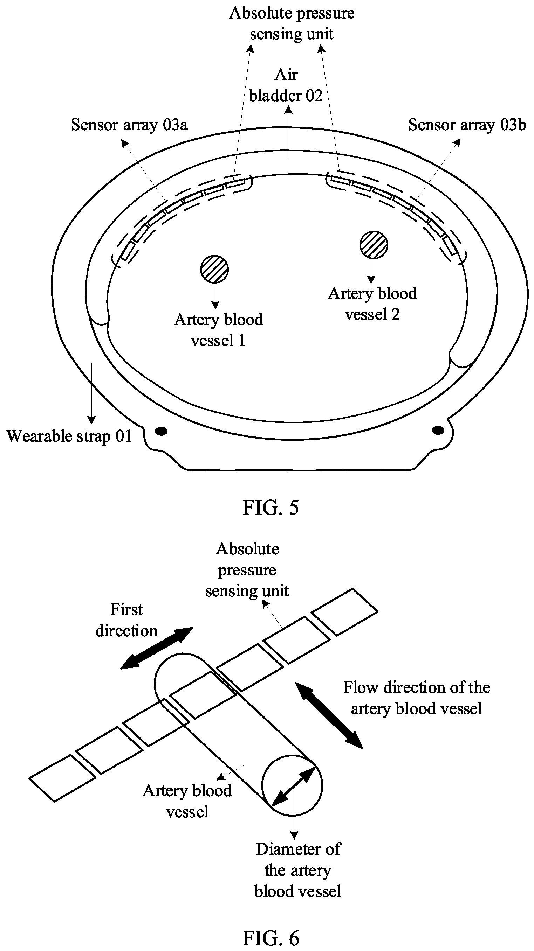

For example, refer to FIG. 4. If the at least one sensor array includes a sensor array 03a and a sensor array 03b, the sensor array 03a and the sensor array 03b are on the side of the air bladder 02 away from the wearable strap 01. In addition, refer to FIG. 5. When the user wears the wearable device, the sensor array 03a corresponds to a position of an artery blood vessel 1 of the user, and the sensor array 03b corresponds to a position of an artery blood vessel 2 of the user.

It should be noted that, when the wearable strap 01 and the air bladder 02 are two independent components, the air bladder 02 is on the inner side of the wearable strap 01. Certainly, in actual application, the wearable strap 01 and the air bladder 02 may alternatively be a whole, and the air bladder 02 is inside the wearable strap. When the user wears the wearable device, the at least one sensor array 03 is on a side that is of the wearable strap 01 and that can contact the skin of the user.

In some embodiments, for any sensor array 03 of the at least one sensor array 03, the sensor array 03 includes a plurality of absolute pressure sensing units, a size of the absolute pressure sensing unit in a first direction is not greater than a diameter of a corresponding artery blood vessel, and the first direction is perpendicular to a flow direction of the corresponding artery blood vessel. The sensor array 03 is configured to collect a pressure experienced by a corresponding artery blood vessel in the process in which the user measures the blood pressure, and the processor is configured to determine the blood pressure of the user based on a pressure data set collected by the at least one sensor array 03.

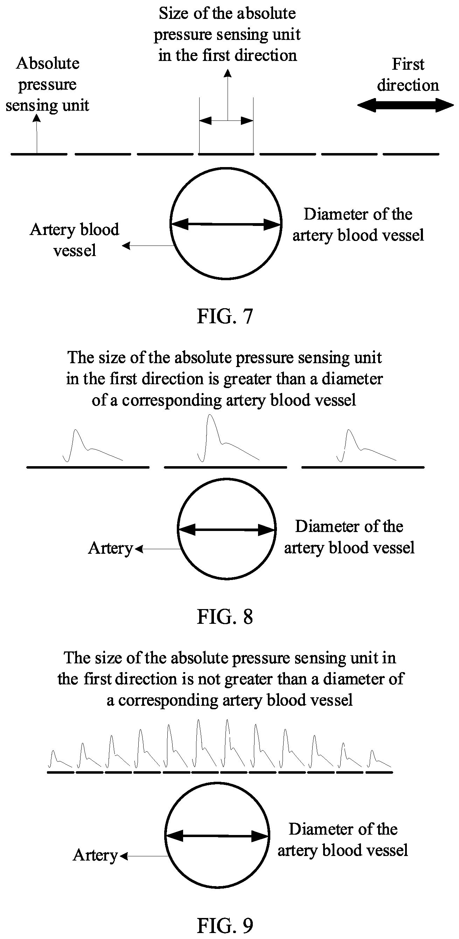

For example, refer to FIG. 6. If the at least one sensor array 03 includes a sensor array a, and the sensor array a includes a plurality of absolute pressure sensing units (in FIG. 6, seven absolute pressure sensing units schematically represent the plurality of absolute pressure sensing units). Refer to FIG. 6 and FIG. 7. The size of the absolute pressure sensing unit in the first direction is not greater than the diameter of the corresponding artery blood vessel, and the first direction is perpendicular to the flow direction of the corresponding artery blood vessel. The sensor array a is configured to collect a pressure experienced by a corresponding artery blood vessel in the process in which the user measures the blood pressure.

In actual application, for an artery blood vessel corresponding to any sensor array, because a distance between an absolute pressure sensing unit right above the artery blood vessel and the artery blood vessel is the smallest, signal strength of a pulse wave right above the artery blood vessel is the largest, and the signal strength of the pulse wave gradually attenuates from right above the artery blood vessel to two sides. Refer to FIG. 8. If the size of the absolute pressure sensing unit in the first direction is greater than the diameter of the corresponding artery blood vessel, a difference between pressure data collected by the absolute pressure sensing units in the sensor array is small, and it is difficult to determine, based on the pressure data collected by the absolute pressure sensing units, the arterial pressure data corresponding to the artery blood vessel. However, in this embodiment of this application, refer to FIG. 9. The size of the absolute pressure sensing unit in the first direction is not greater than the diameter of the corresponding artery blood vessel. In this way, fidelity of signal details of the pressure data set collected by the sensor array is high, so that the arterial pressure data corresponding to the artery blood vessel is conveniently determined, and the finally determined blood pressure is more accurate.

In actual application, a diameter of a standard human artery blood vessel is approximately 2 millimeters to 3 millimeters. In this case, the diameter of the artery blood vessel may be any value between 2 millimeters and 3 millimeters. For example, the diameter of the artery blood vessel may be 3 millimeters. In addition, in different cases, adjustment may be further performed based on different requirements. This is not limited in this embodiment of this application.

In some embodiments, for any sensor array 03, a column direction of the sensor array 03 is the same as a flow direction of the corresponding artery blood vessel, the plurality of absolute pressure sensing units are arranged in a manner of M rows and N columns, a spacing between two adjacent absolute pressure sensing units in a same row is not greater than a diameter of the corresponding artery blood vessel, M is an integer greater than or equal to 1, and N is an integer greater than 1.

It should be noted that an arrangement manner of the plurality of absolute pressure sensing units included in the sensor array 03 is related to an air bladder width of the wearable device, a position and a diameter of an artery blood vessel, and the size of the absolute pressure sensing unit. A skilled person may adjust the arrangement manner of the plurality of absolute pressure sensing units based on an actual requirement. For example, when the size of the absolute pressure sensing unit is 1 millimeter, and the diameter of the artery blood vessel is 3 millimeters, the plurality of absolute pressure sensing units may be arranged in a manner of one row and 24 columns, or may be arranged in a manner of four rows and nine columns, or may be arranged in a manner of four rows and six columns. This is not limited in this embodiment of this application.

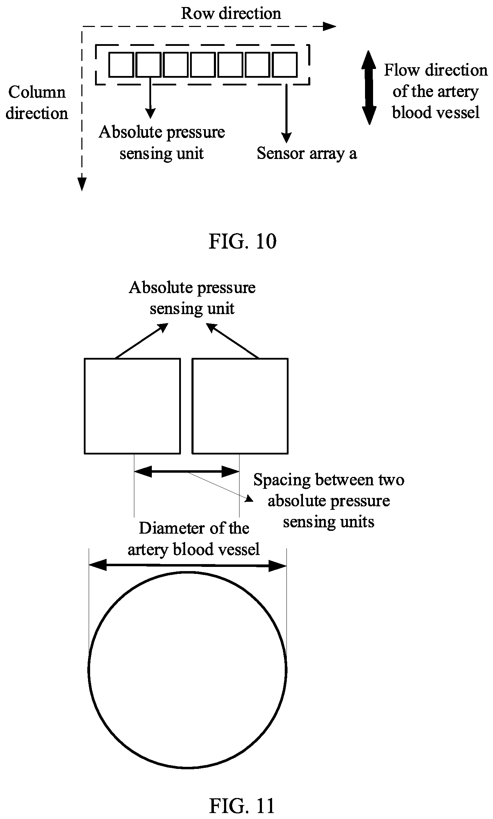

For example, refer to FIG. 10. If the at least one sensor array 03 includes a sensor array a, a column direction of the sensor array a is the same as a flow direction of a corresponding artery blood vessel, and seven absolute pressure sensing units of the sensor array a are arranged in a manner of one row and seven columns. Refer to FIG. 11. A spacing between two adjacent absolute pressure sensing units in a same row in the sensor array a is not greater than a diameter of a corresponding artery blood vessel.

Optionally, for any sensor array 03, absolute pressure sensing units in two adjacent rows of a plurality of absolute pressure sensing units included in the sensor array 03 are staggered.

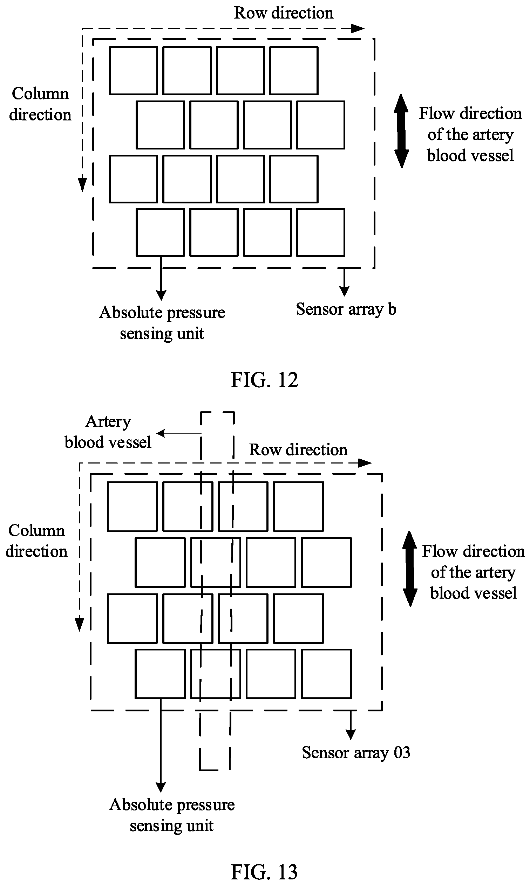

For example, if the at least one sensor array 03 includes a sensor array b, refer to FIG. 12, where absolute pressure sensing units in two adjacent rows of a plurality of absolute pressure sensing units included in the sensor array b are staggered.

Based on the foregoing description, for an artery blood vessel corresponding to any sensor array 03, a pulse wave signal strength collected by an absolute pressure sensing unit right above the artery blood vessel is the largest. However, there is usually a specific spacing between two adjacent absolute pressure sensing units in a same row, and a row direction of the sensor array 03 is perpendicular to a flow direction of a corresponding artery blood vessel. Therefore, refer to FIG. 13. The artery blood vessel may be in a spacing between two adjacent absolute pressure sensing units in an X^{th} row of the sensor array 03, in this case, the two adjacent absolute pressure sensing units cannot be right above the blood vessel, and therefore cannot collect a pulse wave signal with optimal signal strength. However, because absolute pressure sensing units in two adjacent rows of the plurality of absolute pressure sensing units included in the sensor array 03 are staggered, in a previous row and/or a next row of the X^{th} row, there are absolute pressure sensing units right above the artery blood vessel. It can be effectively ensured that the sensor array 03 can collect a pulse wave signal with optimal signal strength, thereby ensuring accuracy of the finally determined blood pressure of the user.

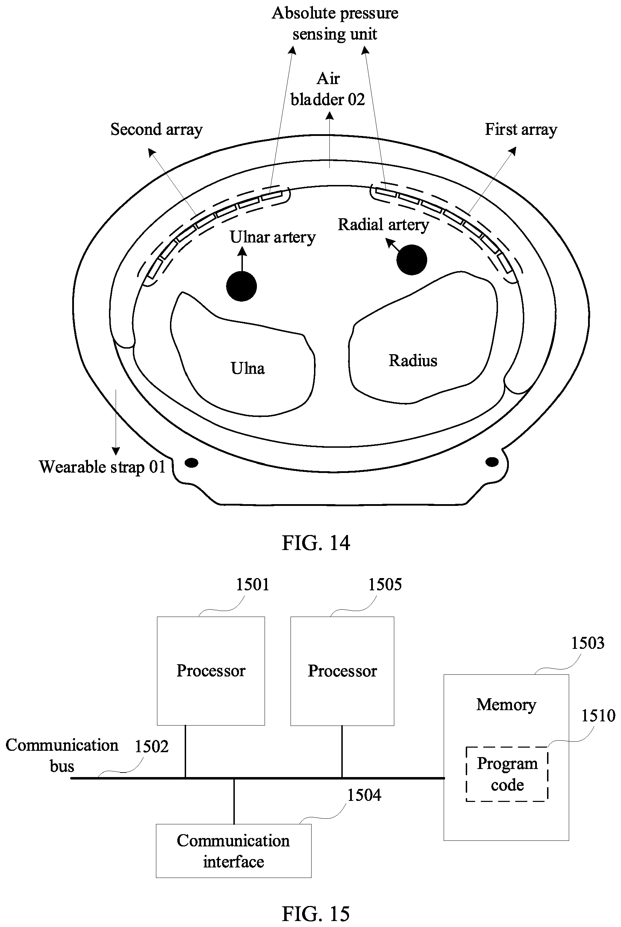

In some embodiments, refer to FIG. 14. The at least one sensor array 03 includes a first array and/or a second array, the first array corresponds to a radial artery blood vessel, and the second array corresponds to an ulnar artery blood vessel.

Optionally, for any sensor array 03, a size of the sensor array 03 in the first direction is greater than three times a diameter of a corresponding artery blood vessel. In other words, the sensor array 03 includes at least three absolute pressure sensing units in the first direction.

It should be noted that, absolute pressure sensing units in two adjacent rows of the plurality of absolute pressure sensing units included in the sensor array may be staggered, or a quantity of absolute pressure sensing units included in each row of the sensor array is different and/or a spacing between adjacent absolute pressure sensing units in a same row is different. In this case, the sensor array is not a regular rectangle, to be specific, the sensor array has a plurality of sizes in the first direction. In this case, that the size of the sensor array 03 in the first direction is greater than three times a diameter of the corresponding artery blood vessel may be understood as that a smallest size of a plurality of sizes of the sensor array in the first direction is greater than three times a diameter of the corresponding artery blood vessel.

In some embodiments, the wearable device can obtain a pressure data set separately collected by at least one sensor array 03; determine, based on the pressure data set collected by the at least one sensor array 03, an effective pressure sensing unit from absolute pressure sensing units included in the at least one sensor array 03; determine, based on the pressure data collected by the effective pressure sensing unit in the at least one sensor array 03, arterial pressure data respectively corresponding to at least one artery blood vessel; and determine a blood pressure of a user based on the arterial pressure data corresponding to the at least one artery blood vessel.

It should be noted that, when the wearable device includes at least two sensor arrays, an arrangement manner of the at least two sensor arrays, a quantity of absolute pressure sensing units in the sensor arrays, and a spacing between the absolute pressure sensing units may be the same or may be different. This is not limited in this embodiment of this application.

The wearable device provided in this embodiment of this application may be worn at any part at which the user can perform blood pressure measurement, for example, a wrist, a leg, or an upper arm. This is not limited in this embodiment of this application. When the wearable device is worn on the wrist of the user to perform blood pressure measurement, the wearable device may be an electronic device that can perform blood pressure measurement, such as a watch, a wristband, or a wrist electronic blood pressure monitor. In this case, the wearable strap may also be referred to as a wrist strap. This is not limited in this embodiment of this application.

A person skilled in the art should understand that the foregoing wearable device is merely an example. Other existing or possible future wearable devices to which embodiments of this application are applicable should also fall within the protection scope of embodiments of this application, and are included herein by reference.

It should be noted that the application scenario and the wearable device that are described in embodiments of this application are intended to describe the technical solutions in embodiments of this application more clearly, and do not constitute a limitation on the technical solutions provided in embodiments of this application. Persons of ordinary skill in the art may be aware that, as a technology evolves and a new application scenario emerges, the technical solutions provided in embodiments of this application are also applicable to a similar technical problem.

FIG. 15 is a diagram of a structure of another wearable device according to an embodiment of this application. The wearable device may be the foregoing wearable device. The wearable device includes at least one processor 1501, a communication bus 1502, a memory 1503, and at least one communication interface 1504.

The processor 1501 may be a general-purpose central processing unit (central processing unit, CPU), a network processor (network processor, NP), or a microprocessor, or may be one or more integrated circuits configured to implement the solutions of this application, for example, an application-specific integrated circuit (application-specific integrated circuit, ASIC), a programmable logic device (programmable logic device, PLD), or a combination thereof. The PLD may be a complex programmable logic device (complex programmable logic device, CPLD), a field-programmable gate array (field-programmable gate array, FPGA), generic array logic (generic array logic, GAL), or any combination thereof.

The communication bus 1502 is configured to transfer information between the foregoing components. The communication bus 1502 may be classified into an address bus, a data bus, a control bus, and the like. For ease of representation, only one thick line is used to represent the bus in the figure, but this does not mean that there is only one bus or only one type of bus.

The memory 1503 may be a read-only memory (read-only memory, ROM), a random access memory (random access memory, RAM), an electrically erasable programmable read-only memory (electrically erasable programmable read-only memory, EEPROM), an optical disc (which includes a compact disc read-only memory (compact disc read-only memory, CD-ROM), a compact disc, a laser disc, a digital versatile disc, a Blu-ray disc, and the like), a magnetic disk storage medium, another magnetic storage device, or any other medium that can be configured to carry or store expected program code in a form of an instruction or a data structure and that can be accessed by a computer, but is not limited thereto. The memory 1503 may exist independently, and is connected to the processor 1501 through the communication bus 1502. Alternatively, the memory 1503 may be integrated with the processor 1501.

In some embodiments, the wearable device may further include at least one communication interface 1504. The communication interface 1504 uses any apparatus such as a transceiver, and is configured to communicate with another device or a communication network. The communication interface 1504 includes a wired communication interface, and may further include a wireless communication interface. The wired communication interface may be, for example, an Ethernet interface. The Ethernet interface may be an optical interface, an electrical interface, or a combination thereof. The wireless communication interface may be a wireless local area network (wireless local area network, WLAN) interface, a cellular network communication interface, a combination thereof, or the like.

In an embodiment, the wearable device may include a plurality of processors, such as the processor 1501 and a processor 1505 shown in FIG. 15. Each of the processors may be a single-core processor, or may be a multicore processor. The processor herein may be one or more devices, circuits, and/or processing cores configured to process data (for example, computer program instructions).

During specific implementation, in an embodiment, the wearable device may further include an output device and an input device. The output device communicates with the processor 1501, and may display information in a plurality of manners. For example, the output device may be a liquid crystal display (liquid crystal display, LCD), a light-emitting diode (light-emitting diode, LED) display device, a cathode ray tube (cathode ray tube, CRT) display device, a projector (projector), or the like. The input device communicates with the processor 1501, and may receive an input from a user in a plurality of manners. For example, the input device may be a touchscreen device or a sensing device.

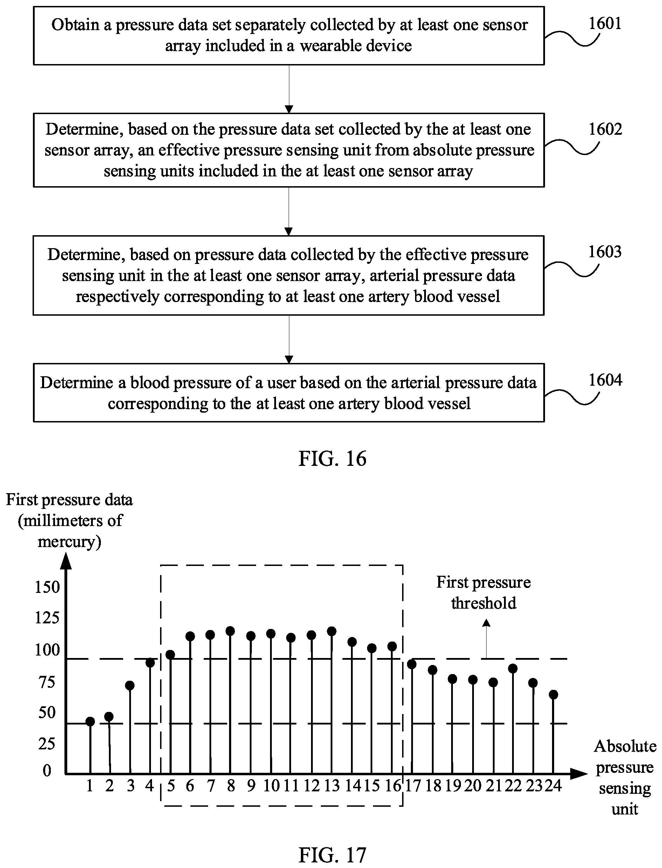

In some embodiments, the memory 1503 is configured to store program code 1510 for executing the solutions of this application, and the processor 1501 may execute the program code 1510 stored in the memory 1503. The program code 1510 may include one or more software modules. The wearable device may implement, by using the processor 1501 and the program code 1510 in the memory 1503, a blood pressure measurement method provided in the following embodiment in FIG. 16.

FIG. 16 is a flowchart of a blood pressure measurement method according to an embodiment of this application. The method is applied to the foregoing wearable device. Refer to FIG. 16. The method includes the following steps.

**Step 1601:** Obtain a pressure data set separately collected by at least one sensor array included in the wearable device, where the pressure data set is collected by a plurality of absolute pressure sensing units included in a corresponding sensor array in a process of inflating and pressurizing an air bladder or deflating and depressurizing the air bladder.

In a process in which the user measures the blood pressure, the air bladder in the wearable device can perform inflation and pressurization at a target rate and then deflation and depressurization, or perform inflation and pressurization and then deflation and depressurization at a target rate, or perform inflation and pressurization at a target rate and then deflation and depressurization at the target rate. In other words, during two processes of inflation and pressurization and deflation and depressurization of the air bladder in the wearable device, there is at least one process in which the pressure changes at the target rate, and the at least one sensor array can collect a pressure data set during the process in which the pressure of the air bladder changes at the target rate.

The target rate is set in advance. An upper limit of a value range of the target rate is related to a sampling frequency of the sensor array and a heart rate of the user. A higher sampling frequency of the sensor array and/or a higher heart rate of the user indicate a higher upper limit of the value range of the target rate. In other words, the sampling frequency of the sensor array and the heart rate of the user are in direct proportion to the upper limit of the value range of the target rate.

**Step 1602:** Determine, based on the pressure data set collected by the at least one sensor array, an effective pressure sensing unit from absolute pressure sensing units included in the at least one sensor array, where pressure data collected by the effective pressure sensing unit is able to effectively represent a pulsation status of an artery blood vessel of a user.

In some embodiments, the pressure data set includes a plurality of groups of pressure data collected by a plurality of absolute pressure sensing units included in a corresponding sensor array, and each group of pressure data includes pressure data at a plurality of moments.

In other words, for any sensor array, a pressure data set collected by the sensor array includes a plurality of groups of pressure data, the plurality of groups of pressure data are in a one-to-one correspondence with a plurality of absolute pressure sensing units included in the sensor array, and each group of pressure data is pressure data separately collected by a corresponding absolute pressure sensing unit at a plurality of moments.

In a possible implementation, the wearable device determines, based on a first pressure data set, a plurality of groups of candidate pressure sensing units from absolute pressure sensing units included in a first sensor array, and determines an effective pressure sensing unit in the first sensor array from the plurality of groups of candidate pressure sensing units.

The first pressure data set is a pressure data set collected by the first sensor array, the first sensor array is any one of the at least one sensor array included in the wearable device, the plurality of groups of candidate pressure sensing units are in a one-to-one correspondence with a plurality of first moments, positions of a same group of candidate pressure sensing units are consecutive, and a difference between pressure data collected by the same group of candidate pressure sensing units at a corresponding first moment falls within a pressure fluctuation range, where the first moment is one of the plurality of moments, and the first artery blood vessel is an artery blood vessel corresponding to the first sensor array.

In some embodiments, the wearable device stores coordinates of each absolute pressure sensing unit in the first sensor array. In this case, that the positions of the same group of candidate pressure sensing units are consecutive means that horizontal coordinates and/or vertical coordinates of the same group of candidate pressure sensing units are consecutive. In addition, if a difference between pressure data collected by the same group of candidate pressure sensing units at a same moment falls within a pressure fluctuation range, it indicates that the difference between the pressure data collected by the same group of candidate pressure sensing units is small.

It should be noted that the pressure fluctuation range is preset. For example, the pressure fluctuation range may be set to -5 millimeters of mercury to 5 millimeters of mercury, and may be adjusted based on different requirements in different cases.

In actual application, a pressure at a position at which the air bladder contacts the skin is far greater than a pressure at a position at which the air bladder does not contact the skin, and a difference between pressure data at the position at which the air bladder contacts the skin is small. Therefore, if positions of the same group of candidate pressure sensing units are consecutive and a difference between collected pressure data is small, it indicates that the group of candidate pressure sensing units may be at a position at which the air bladder contacts the skin. In this case, the pressure data collected by the group of candidate pressure sensing units is effective. In this way, accuracy and reliability of subsequently determined blood pressure of the user can be ensured.

An implementation process of determining, based on the first pressure data set, the plurality of groups of candidate pressure sensing units from the absolute pressure sensing units included in the first sensor array includes: determining a plurality of first moments from a plurality of moments, and for any first moment of the plurality of first moments, determining, based on a plurality of pieces of first pressure data and positions of a plurality of absolute pressure sensing units included in the first sensor array, a group of candidate pressure sensing units corresponding to the first moment from the absolute pressure sensing units included in the first sensor array, where the plurality of pieces of first pressure data are in a one-to-one correspondence with the plurality of absolute pressure sensing units included in the first sensor array; and the first pressure data is pressure data collected by a corresponding absolute pressure sensing unit at the first moment. Each first moment in the plurality of first moments is processed in a same manner, so that candidate pressure sensing units respectively corresponding to the plurality of first moments can be determined.

It should be noted that the pressure data collected by the absolute pressure sensing unit at the first moment includes a static pressure and a dynamic pressure. The static pressure represents a pressure applied by the air bladder at the first moment, and the dynamic pressure represents a pulsation status of a corresponding artery blood vessel under compression of the static pressure. In this case, the first pressure data may alternatively be a static pressure collected by the corresponding absolute pressure sensing unit at the first moment. In other words, the pressure data (original pressure data) collected by the absolute pressure sensing unit at the first moment includes the static pressure and the dynamic pressure at the first moment. In this case, the first pressure data may be the original pressure data collected at the first moment, or may be the static pressure at the first moment. This is not limited in this embodiment of this application.

Because the first pressure data set may be collected in a process of inflating and pressurizing the air bladder, or may be collected in a process of deflating and depressurizing the air bladder, if the first pressure data set is collected in the process of inflating and pressurizing the air bladder, at least one piece of pressure data whose pressure data is a reference pressure value may be determined from the first pressure data set, an earliest moment corresponding to the at least one piece of pressure data is used as a first start moment, and moments that are in the plurality of moments and that are later than the first start moment are used as the plurality of first moments. If the first pressure data set is collected in the process of deflating and depressurizing the air bladder, at least one piece of pressure data whose pressure data is a reference pressure value may be determined from the first pressure data set, a latest moment corresponding to the at least one piece of pressure data is used as the first start moment, and moments that are in the plurality of moments and that are earlier than the first start moment are used as the plurality of first moments. Certainly, in actual application, the plurality of first moments may be determined from the plurality of moments in another manner. For example, some or all of the plurality of moments may be directly used as the plurality of first moments. This is not limited in this embodiment of this application.

In actual application, when a pressure applied by the air bladder on the artery blood vessel is excessively small or excessively large, the absolute pressure sensing unit cannot clearly collect pulse wave data of the artery blood vessel. Therefore, the reference pressure value is a pressure value at which the absolute pressure sensing unit can clearly collect the pulse wave data of the artery blood vessel. The reference pressure value is set by a skilled person based on experience. For example, the reference pressure value may be set to 80 millimeters of mercury, and in different cases, adjustment may be further performed based on different requirements.

Because the reference pressure value is a pressure value at which the absolute pressure sensing unit can clearly collect the pulse wave data of the artery blood vessel, if pressure data collected by the absolute pressure sensing unit in the first sensor array reaches the reference pressure value, it indicates that an absolute pressure sensing unit that is in the first sensor array and that is close to the artery blood vessel can collect a pulse wave signal. In this case, accuracy of the determined candidate pressure sensing unit can be ensured.

Optionally, an implementation process of determining, based on the plurality of pieces of first pressure data and the positions of the plurality of absolute pressure sensing units included in the first sensor array, a group of candidate pressure sensing units corresponding to the first moment from the absolute pressure sensing units included in the first sensor array includes: determining, based on the plurality of pieces of first pressure data and the positions of the plurality of absolute pressure sensing units included in the first sensor array, at least one group of first pressure sensing units from the absolute pressure sensing units included in the first sensor array, where positions of a same group of first pressure sensing units are consecutive and a difference between collected first pressure data falls within a pressure fluctuation range; and selecting, based on the first pressure data of the at least one group of first pressure sensing units, a group of first pressure sensing units as the group of candidate pressure sensing units corresponding to the first moment.

In a possible implementation, a first mean value respectively corresponding to each group of first pressure sensing units is determined based on the first pressure data of the group of first pressure sensing units, to obtain at least one first mean value, where the first mean value is a mean value of first pressure data of a corresponding group of first pressure sensing units; and a group of first pressure sensing units corresponding to a largest first mean value of the at least one first mean value is used as a target pressure sensing unit group, and in the target pressure sensing unit group, performing deletion processing on a first pressure sensing unit whose first pressure data is less than a first pressure threshold, and using a target pressure sensing unit group obtained after the deletion processing as the group of candidate pressure sensing units corresponding to the first moment, where the first pressure threshold is a product of a first mean value corresponding to the target pressure sensing unit group and a pressure proportion.

The pressure proportion is preset. For example, the pressure proportion may be set to 75 percent. In addition, in different cases, adjustment may be further performed based on different requirements.

For example, if the first sensor array includes 48 absolute pressure sensing units, the 48 absolute pressure sensing units are arranged in a manner of 24 rows and 2 columns, and the 48 absolute pressure sensing units are respectively absolute pressure sensing units 1-24 corresponding to the first column and absolute pressure sensing units 1-24 corresponding to the second column. The absolute pressure sensing units 1-24 corresponding to the first column are used as an example. Refer to FIG. 17. FIG. 17 shows first pressure data collected by 24 absolute pressure sensing units at a moment A of a plurality of first moments. Horizontal coordinates in FIG. 17 are the absolute pressure sensing units 1-24, and vertical coordinates are the first pressure data. It can be easily seen from FIG. 17 that the first pressure data of the absolute pressure sensing units 5-16 is greater than the first pressure threshold. Therefore, the absolute pressure sensing units 5-16 are a group of candidate pressure sensing units corresponding to the moment a.

In some embodiments, an implementation process of determining the effective pressure sensing unit in the first sensor array from the plurality of groups of candidate pressure sensing units includes: determining at least one first sensing unit from a plurality of groups of candidate pressure sensing units, determining, based on the first pressure data set, at least one second sensing unit from absolute pressure sensing units included in the first sensor array, where positions of the at least one second sensing unit are consecutive, and using an intersection set of the at least one first sensing unit and the at least one second sensing unit as the effective pressure sensing unit in the first sensor array.

It should be noted that, that the positions of the at least one second sensing unit are consecutive means that horizontal coordinates of the at least one second sensing unit are consecutive and/or vertical coordinates of the at least one second sensing unit are consecutive.

Optionally, each candidate pressure sensing unit in the plurality of groups of candidate pressure sensing units or an intersection set of the plurality of groups of candidate pressure sensing units may be used as the at least one first sensing unit. Certainly, in actual application, the at least one first sensing unit may be determined in another manner. This is not limited in this embodiment of this application.

In some embodiments, the first pressure data set includes pressure data collected by each absolute pressure sensing unit in the first sensor array, the pressure data includes a static pressure and a dynamic pressure corresponding to the static pressure, the static pressure represents a pressure applied by the air bladder, and the dynamic pressure represents a pulsation status of a corresponding artery blood vessel under compression of the static pressure. In this case, based on a dynamic pressure collected by each absolute pressure sensing unit in the first sensor array, a maximum peak-to-peak value corresponding to each absolute pressure sensing unit in the first sensor array is determined to obtain a plurality of maximum peak-to-peak values; at least one second candidate sensing unit is determined based on the plurality of maximum peak-to-peak values, where the at least one second candidate sensing unit is consecutive in position, and a difference between corresponding maximum peak-to-peak values is within a peak-to-peak fluctuation range; and at least one second sensing unit is determined based on the at least one second candidate sensing unit.

The peak-to-peak value fluctuation range is preset, and may be adjusted based on different requirements in different cases.

For any absolute pressure sensing unit in the first sensor array, a plurality of peak-to-peak values of a dynamic pressure of the absolute pressure sensing unit are determined, where the plurality of peak-to-peak values are in a one-to-one correspondence with a plurality of arterial pulsation cycles included in the dynamic pressure, and a maximum peak-to-peak value of the plurality of peak-to-peak values is used as a maximum peak-to-peak value corresponding to the absolute pressure sensing unit.

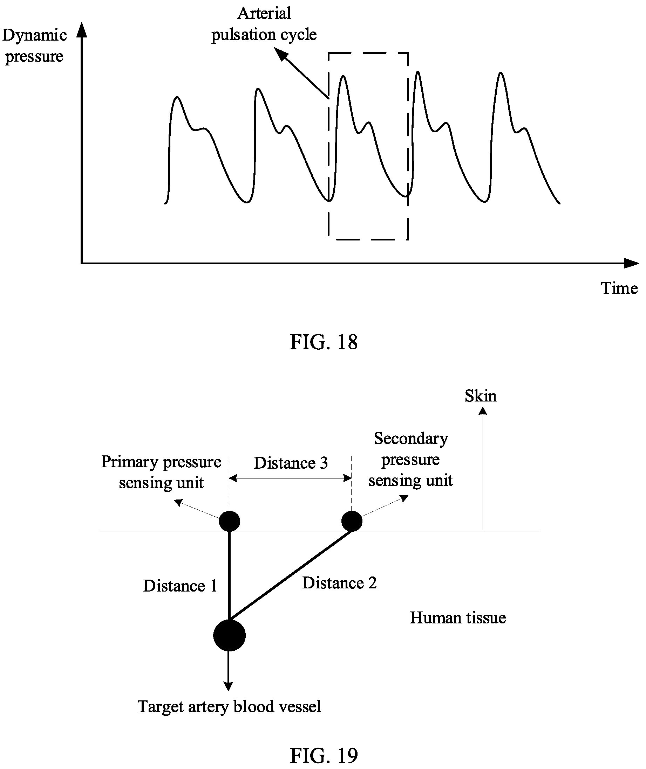

It should be noted that the dynamic pressure collected by the effective pressure sensing unit is actually a pulse wave signal of an artery blood vessel. FIG. 18 is a diagram of a pulse wave signal of an artery blood vessel. The pulse wave signal includes a plurality of arterial pulsation cycles, and a peak-to-peak value of the dynamic pressure is a difference between a maximum dynamic pressure and a minimum dynamic pressure in one arterial pulsation cycle.

In some embodiments, the at least one second candidate sensing unit may be directly determined as the at least one second sensing unit. In some other embodiments, for the at least one second candidate sensing unit, deletion processing may also be performed on a second candidate sensing unit whose maximum peak-to-peak value is smaller than a maximum peak-to-peak value threshold, and the at least one second candidate sensing unit obtained after the deletion processing is used as the at least one second candidate sensing unit, where the maximum peak-to-peak value threshold is a product of a mean value of maximum peak-to-peak values corresponding to the at least one second candidate sensing unit and a peak-to-peak value ratio.

The peak-to-peak value ratio is preset. For example, the peak-to-peak value ratio may be set to 75 percent. In addition, in different cases, adjustment may be further performed based on different requirements.

Based on the foregoing description, for an artery blood vessel corresponding to any sensor array, signal strength of a pulse wave right above the artery blood vessel is the largest, and the signal strength of the pulse wave gradually attenuates from right above the artery blood vessel to two sides. Therefore, if positions of at least one second candidate sensing unit are consecutive and a difference between corresponding maximum peak-to-peak values is small, it indicates that the at least one second candidate sensing unit may be around the artery blood vessel. In this case, only pressure data collected by the at least one second candidate sensing unit is effective, or in other words, data collected by the group of candidate pressure sensing units is pressure data that can represent artery blood vessel pulsation. In this way, accuracy and reliability of subsequently determined blood pressure of the user can be ensured.

In addition, because the first sensing unit is at the position at which the air bladder contacts the skin, the second sensing unit is around the artery blood vessel, and the effective pressure sensing unit in the first sensor array is an intersection set of the at least one first sensing unit and the at least one second sensing unit, the effective pressure sensing unit is an absolute pressure sensing unit at the position at which the air bladder contacts the skin and around the artery blood vessel. In this case, arterial pressure data determined in a subsequent step based on the pressure data collected by the effective pressure sensing unit is accurate and effective, thereby further ensuring accuracy and reliability of the subsequently determined blood pressure of the user.

In conclusion, in this embodiment of this application, an absolute pressure sensing unit at a position at which the air bladder contacts the skin can be determined based on the pressure data collected by the first sensor array, and an absolute pressure sensing unit around an artery blood vessel can be further determined based on the dynamic pressure collected by the first sensor array, to determine an effective pressure sensing unit at the position at which the air bladder contacts the skin and around the artery blood vessel. Certainly, in actual application, if the first sensor array has a small quantity of rows, the absolute pressure sensing unit at the position at which the air bladder contacts the skin in the first sensor array may not need to be determined, only the absolute pressure sensing unit around the artery blood vessel needs to be determined based on the dynamic pressure collected by the first sensor array, and the absolute pressure sensing unit around the artery blood vessel is used as the effective pressure sensing unit.

If the first sensor array has a small quantity of rows, it indicates that a width of the first sensor array needs to be less than the width of the air bladder in the wearable device. In this case, it may be considered that all absolute pressure sensing units in the first sensor array are at the position at which the air bladder contacts the skin. Therefore, the absolute pressure sensing unit around the artery blood vessel may be directly determined based on the dynamic pressure collected by the first sensor array, and the absolute pressure sensing unit around the artery blood vessel is used as the effective pressure sensing unit.

**Step 1603:** Determine, based on the pressure data collected by the effective pressure sensing unit in the at least one sensor array, arterial pressure data respectively corresponding to the at least one artery blood vessel, where the arterial pressure data represents a pressure actually experienced by a corresponding artery blood vessel in a blood pressure measurement process.

The arterial pressure data corresponding to the second artery blood vessel is determined based on the pressure data collected by the effective pressure sensing unit in the second sensor array, where the second sensor array is any one of the at least one sensor array, and the second artery blood vessel is an artery blood vessel corresponding to the second sensor array.

There are a plurality of implementations of determining, based on the pressure data collected by the effective pressure sensing unit in the second sensor array, the arterial pressure data corresponding to the second artery blood vessel. The following describes two implementations.

**In a first implementation,** the pressure data is pressure data separately collected by the effective pressure sensing unit at a plurality of moments, and the arterial pressure data is arterial pressure data respectively corresponding to the plurality of moments. In this case, for any one of the plurality of moments, a mean value or a maximum value of pressure data collected by the effective pressure sensing unit at the moment is used as arterial pressure data corresponding to the moment, and the arterial pressure data corresponding to the plurality of moments can be obtained in a same manner.

**In a second implementation,** pressure data collected by an effective pressure sensing unit in the second sensor array is determined as the arterial pressure data corresponding to the second artery blood vessel.

Optionally, a maximum peak-to-peak value corresponding to each effective pressure sensing unit is determined based on pressure data collected by the effective pressure sensing unit in the second sensor array, to obtain at least one maximum peak-to-peak value, and pressure data collected by an effective pressure sensing unit corresponding to a largest maximum peak-to-peak value in the at least one maximum peak-to-peak value is used as the arterial pressure data corresponding to the second artery blood vessel.

Optionally, the pressure data is pressure data separately collected by the effective pressure sensing unit at a plurality of moments, the pressure data includes a static pressure and a dynamic pressure corresponding to the static pressure, the static pressure represents a pressure applied by the air bladder, and the dynamic pressure represents a pulsation status of a corresponding artery blood vessel under compression of the static pressure. In other words, the pressure data includes a plurality of static pressures and a plurality of dynamic pressures, and the plurality of static pressures and the plurality of dynamic pressures are in a one-to-one correspondence with the plurality of moments.

In this case, an implementation process of determining, based on the pressure data collected by the effective pressure sensing units in the second sensor array, the maximum peak-to-peak value corresponding to each effective pressure sensing unit includes: for any effective pressure sensing unit in the second sensor array, determining a plurality of peak-to-peak values of a dynamic pressure of the effective pressure sensing unit, where the plurality of peak-to-peak values are in a one-to-one correspondence with a plurality of arterial pulsation cycles included in the dynamic pressure, and a maximum peak-to-peak value of the plurality of peak-to-peak values is used as a maximum peak-to-peak value corresponding to the effective pressure sensing unit. The effective pressure sensing units in the second sensor array are processed in a same manner, to determine a maximum peak-to-peak value corresponding to each effective pressure sensing unit. Certainly, in actual application, the maximum peak-to-peak value corresponding to each effective pressure sensing unit may be determined in another manner. This is not limited in this embodiment of this application.

Based on the foregoing description, the wearable device may use, as the arterial pressure data corresponding to the second artery blood vessel, pressure data collected by an effective pressure sensing unit that corresponds to a largest MAP in the effective pressure sensing units. Certainly, in actual application, pressure data collected by any effective pressure sensing unit in the second sensor array may be alternatively determined as the arterial pressure data corresponding to the second artery blood vessel.

In actual application, before determining, based on the pressure data collected by the effective pressure sensing unit in the at least one sensor array, the arterial pressure data respectively corresponding to the at least one artery blood vessel, the wearable device may further determine a tissue attenuation coefficient corresponding to at least one effective pressure sensing unit in a target sensor array, where the tissue attenuation coefficient indicates an attenuation status of a pulse wave of a target artery blood vessel caused by a human tissue, the target artery blood vessel is an artery blood vessel corresponding to the target sensor array, and the target sensor array is any sensor array in the at least one sensor array; and correct, based on the tissue attenuation coefficient corresponding to the at least one effective pressure sensing unit, pressure data collected by the at least one effective pressure sensing unit.

In other words, in this embodiment of this application, considering attenuation of the pulse wave of the target artery blood vessel by the human tissue, the pressure data collected by the effective pressure sensing unit is corrected by calculating the tissue attenuation coefficient, so that the corrected pressure data collected by the effective pressure sensing unit can accurately reflect a pulse of the artery blood vessel, thereby ensuring accuracy and reliability of finally determined blood pressure of the user.

In some embodiments, an implementation process of determining a tissue attenuation coefficient corresponding to at least one effective pressure sensing unit in the target sensor array includes: determining a distance between the at least one effective pressure sensing unit and the target artery blood vessel, and determining a unit attenuation coefficient, where the unit attenuation coefficient is an attenuation status of the pulse wave of the target artery blood vessel caused by a human tissue with a unit thickness; and determining, based on the unit attenuation coefficient and the distance between the at least one effective pressure sensing unit and the target artery blood vessel, the tissue attenuation coefficient respectively corresponding to the at least one effective pressure sensing unit.

Optionally, a distance between a first effective pressure sensing unit and the target artery blood vessel is determined, and a unit attenuation coefficient is determined. The first effective pressure sensing unit is any effective pressure sensing unit in the at least one effective pressure sensing unit, and a value obtained by multiplying the unit attenuation coefficient by the distance between the first effective pressure sensing unit and the target artery blood vessel is used as a tissue attenuation coefficient corresponding to the first effective pressure sensing unit. Each first effective pressure sensing unit in the at least one first effective pressure sensing unit is processed in a same manner, so that the tissue attenuation coefficient respectively corresponding to the at least one effective pressure sensing unit can be determined.

In some embodiments, a primary pressure sensing unit and a secondary pressure sensing unit in the effective sensing units of the target sensor array are determined based on pressure data collected by the effective pressure sensing units in the target sensor array, where the primary pressure sensing unit is an absolute pressure sensing unit with highest signal strength in the effective sensing units of the target sensor array, and signal strength of the secondary pressure sensing unit is less than signal strength of the primary pressure sensing unit, and based on the primary pressure sensing unit and the secondary pressure sensing unit in the effective sensing units, a distance between the first effective pressure sensing unit and the target artery blood vessel is determined, and the unit attenuation coefficient is determined.

Optionally, a maximum peak-to-peak value corresponding to each effective pressure sensing unit is determined based on pressure data collected by the effective pressure sensing unit in the target sensor array, to obtain at least one maximum peak-to-peak value, and an effective pressure sensing unit corresponding to a largest maximum peak-to-peak value in the at least one maximum peak-to-peak value is used as the primary pressure sensing unit. An effective pressure sensing unit corresponding to a maximum peak-to-peak value, in the at least one maximum peak-to-peak value, that is not equal to the largest maximum peak-to-peak value and for which a difference is greater than a maximum peak-to-peak value difference threshold, is used as the secondary pressure sensing unit.

Certainly, in actual application, the primary pressure sensing unit and the secondary pressure sensing unit may alternatively be determined in another manner. For example, an effective pressure sensing unit with a maximum mean value of pressure data collected by the effective pressure sensing units in the target sensor array is used as the primary pressure sensing unit. For another example, an effective pressure sensing unit other than the primary pressure sensing unit in the effective pressure sensing units of the target sensor array is directly used as the secondary pressure sensing unit. This is not limited in this embodiment of this application.

The maximum peak-to-peak value difference threshold is preset. For example, the maximum peak-to-peak value difference threshold may be set to 2, and may be further adjusted based on requirements in different cases.

It should be noted that the foregoing implementation of determining, based on the pressure data collected by the effective pressure sensing units in the target sensor array, the maximum peak-to-peak value corresponding to each effective pressure sensing unit is similar to the foregoing implementation of determining, based on the pressure data collected by the effective pressure sensing units in the second sensor array, the maximum peak-to-peak value corresponding to each effective pressure sensing unit. For detailed content, refer to the foregoing related content. Details are not described herein again.

In actual application, the air bladder included in the wearable device is a narrow air bladder, and the wearable device further includes a barometric pressure sensor. The barometric pressure sensor is configured to collect a gas pressure inside the air bladder. In this case, after the primary pressure sensing unit in the effective sensing units is determined, a mean arterial pressure (mean arterial pressure, MAP) corresponding to the primary pressure sensing unit may be further determined based on the pressure data collected by the primary pressure sensing unit, and a MAP corresponding to the barometric pressure sensor is determined based on the pressure data collected by the barometric pressure sensor and according to a related algorithm. If the MAP corresponding to the primary pressure sensing unit is greater than or equal to the MAP corresponding to the barometric pressure sensor, step 1601 is performed again, in other words, the blood pressure of the user is re-measured, and if the MAP corresponding to the primary pressure sensing unit is less than the MAP corresponding to the barometric pressure sensor, the foregoing step of determining the secondary pressure sensing unit continues to be performed.

Based on the foregoing description, when the narrow air bladder is inflated, the pressure data collected by the barometric pressure sensor is higher than the pressure actually experienced by the artery blood vessel. Therefore, in a normal case, the MAP corresponding to the primary pressure sensing unit should be less than the MAP corresponding to the barometric pressure sensor. If the MAP corresponding to the primary pressure sensing unit is greater than or equal to the MAP corresponding to the barometric pressure sensor, it indicates that a measurement result of the wearable device is incorrect, and measurement needs to be performed again.

In some embodiments, the pressure data collected by the primary pressure sensing unit is pressure data separately collected by the primary pressure sensing unit at a plurality of moments, and the pressure data includes a static pressure and a dynamic pressure corresponding to the static pressure. In this case, a static pressure corresponding to a first dynamic pressure in dynamic pressures of the primary pressure sensing unit is used as the MAP corresponding to the primary pressure sensing unit, where the first dynamic pressure is a dynamic pressure corresponding to a maximum peak-to-peak value in the dynamic pressures of the primary pressure sensing unit.

Based on the foregoing description, the peak-to-peak value of the dynamic pressure is a difference between a maximum dynamic pressure and a minimum dynamic pressure in one arterial pulsation cycle. Therefore, the dynamic pressure corresponding to the maximum peak-to-peak value is either of the maximum dynamic pressure and the minimum dynamic pressure that are corresponding to the maximum peak-to-peak value in a corresponding arterial pulsation cycle, or is a higher dynamic pressure in the two dynamic pressures, or is a lower dynamic pressure in the two dynamic pressures, or is a mean value of the maximum peak-to-peak value in the corresponding arterial pulsation cycle, or is a dynamic pressure corresponding to a time midpoint of the corresponding arterial pulsation cycle, or the like. Certainly, in actual application, the dynamic pressure corresponding to the maximum peak-to-peak value may be alternatively determined in another manner. This is not limited in this embodiment of this application.

If the first effective pressure sensing unit is the primary pressure sensing unit, an implementation of determining the distance between the first effective pressure sensing unit and the target artery blood vessel and determining the unit attenuation coefficient is different from that of determining the first effective pressure sensing unit as the secondary pressure sensing unit, which are separately described below.

**If the first effective pressure sensing unit is the primary pressure sensing unit,** the distance between the first effective pressure sensing unit and the target artery blood vessel and a distance between a target secondary pressure sensing unit and the target artery blood vessel are determined based on the pressure data collected by the first effective pressure sensing unit, pressure data collected by the target secondary pressure sensing unit, and a distance between the first effective pressure sensing unit and the target secondary pressure sensing unit in the first direction, where the target secondary pressure sensing unit is any secondary pressure sensing unit in the target sensor array, and the unit attenuation coefficient is determined based on the pressure data collected by the first effective pressure sensing unit, the pressure data collected by the target secondary pressure sensing unit, the distance between the first effective pressure sensing unit and the target artery blood vessel, and the distance between the target secondary pressure sensing unit and the target artery blood vessel.

In some embodiments, the wearable device stores a distance between any two absolute pressure sensing units in the target sensor array in the first direction. If the first effective pressure sensing unit is the primary pressure sensing unit, the wearable device can determine a distance between the first effective pressure sensing unit and the target artery blood vessel and a distance between a target secondary pressure sensing unit and the target artery blood vessel based on a related formula such as a trigonometric function based on a ratio of a MAP corresponding to the target secondary pressure sensing unit to a MAP corresponding to the first effective pressure sensing unit and a distance between the first effective pressure sensing unit and the target secondary pressure sensing unit in the first direction.

It should be noted that an implementation of determining the MAP corresponding to the target secondary pressure sensing unit is similar to the foregoing implementation of determining the MAP corresponding to the primary pressure sensing unit. For detailed content, refer to the foregoing related content. Details are not described herein again.

Based on the foregoing description, the absolute pressure sensing unit right above the artery blood vessel collects a pulse wave with highest signal strength, and the primary pressure sensing unit is an absolute pressure sensing unit with highest signal strength in the effective sensing units of the target sensor array. Refer to FIG. 19. The primary pressure sensing unit may be considered as an absolute pressure sensing unit right above the target artery blood vessel in the target sensor array, and a distance between the primary pressure sensing unit and the target artery blood vessel is a distance 1. The secondary pressure sensing unit is an absolute pressure sensing unit far away from the target artery blood vessel. A distance between the secondary pressure sensing unit and the target artery blood vessel is a distance 2. A ratio of a MAP corresponding to the secondary pressure sensing unit to a MAP corresponding to the primary pressure sensing unit can represent a ratio of the distance 1 to the distance 2. Therefore, based on the ratio of the MAP corresponding to the secondary pressure sensing unit to the MAP corresponding to the primary pressure sensing unit, and a distance (that is, a distance 3 in FIG. 19) between the primary pressure sensing unit and the secondary pressure sensing unit in the first direction, the distance 1 and the distance 3 can be determined based on a related formula such as a trigonometric function.

In some other embodiments, if the first effective pressure sensing unit is the primary pressure sensing unit, the wearable device may alternatively determine a distance between the first effective pressure sensing unit and the target artery blood vessel and a distance between a target secondary pressure sensing unit and the target artery blood vessel based on a related formula such as a trigonometric function based on a ratio of a MAP corresponding to the target secondary pressure sensing unit to a MAP corresponding to the first effective pressure sensing unit, a maximum peak-to-peak value corresponding to the target secondary pressure sensing unit, a maximum peak-to-peak value corresponding to the first effective pressure sensing unit, and a distance between the first effective pressure sensing unit and the target secondary pressure sensing unit in the first direction. Certainly, in actual application, the distance between the first effective pressure sensing unit and the target artery blood vessel and the distance between the target secondary pressure sensing unit and the target artery blood vessel may be alternatively determined in another manner. This is not limited in this embodiment of this application.

In actual application, in a pulse wave signal propagation process, not all human tissues between the absolute pressure sensing unit and the target artery blood vessel attenuate the signal, and a human tissue of a specific thickness does not affect the signal strength. The thickness of the human tissue that does not attenuate the signal is related to factors such as age, fatness, and gender of the user. Therefore, in some embodiments, the wearable device may further correct the distance between the first effective pressure sensing unit and the target artery blood vessel and the distance between the target secondary pressure sensing unit and the target artery blood vessel, that is, obtain basic information of the user, where the basic information includes at least one of gender, height data, and weight data; and correct the distance between the first effective pressure sensing unit and the target artery blood vessel and the distance between the target secondary pressure sensing unit and the target artery blood vessel based on the basic information.

Optionally, the user may enter at least one of gender, height data, and weight data of the user into the wearable device, so that the wearable device can obtain the basic information of the user.

An implementation process of determining the unit attenuation coefficient based on the pressure data collected by the first effective pressure sensing unit, the pressure data collected by the target secondary pressure sensing unit, the distance between the first effective pressure sensing unit and the target artery blood vessel, and the distance between the target secondary pressure sensing unit and the target artery blood vessel includes: dividing a MAP difference by a distance difference to obtain the unit attenuation coefficient, where the MAP difference is a difference between a MAP of the first effective pressure sensing unit and a MAP of the target secondary pressure sensing unit, the distance difference is a difference between a first distance and a second distance, the first distance is the distance between the first effective pressure sensing unit and the target artery blood vessel, and the second distance is the distance between the target secondary pressure sensing unit and the target artery blood vessel.

**If the first effective pressure sensing unit is the secondary pressure sensing unit,** the distance between the first effective pressure sensing unit and the target artery blood vessel and a distance between the primary pressure sensing unit and the target artery blood vessel are determined based on the pressure data collected by the first effective pressure sensing unit, pressure data collected by the primary pressure sensing unit, and a distance between the first effective pressure sensing unit and the primary pressure sensing unit in the first direction; and the unit attenuation coefficient is determined based on the pressure data collected by the first effective pressure sensing unit, the pressure data collected by the primary pressure sensing unit, the distance between the first effective pressure sensing unit and the target artery blood vessel, and the distance between the primary pressure sensing unit and the target artery blood vessel.

An implementation of determining the distance between the first effective pressure sensing unit and the target artery blood vessel and the distance between the primary pressure sensing unit and the target artery blood vessel based on the pressure data collected by the first effective pressure sensing unit, the pressure data collected by the primary pressure sensing unit, and the distance between the first effective pressure sensing unit and the primary pressure sensing unit in the first direction is similar to the foregoing implementation of determining the distance between the first effective pressure sensing unit and the target artery blood vessel and the distance between the target secondary pressure sensing unit and the target artery blood vessel based on the pressure data collected by the first effective pressure sensing unit, the pressure data collected by the target secondary pressure sensing unit, and the distance between the first effective pressure sensing unit and the target secondary pressure sensing unit in the first direction. For detailed content, refer to the foregoing related content. Details are not described herein again.

An implementation of determining the unit attenuation coefficient based on the pressure data collected by the first effective pressure sensing unit, the pressure data collected by the primary pressure sensing unit, the distance between the first effective pressure sensing unit and the target artery blood vessel, and the distance between the primary pressure sensing unit and the target artery blood vessel is similar to the foregoing implementation of determining the unit attenuation coefficient based on the pressure data collected by the first effective pressure sensing unit, the pressure data collected by the target secondary pressure sensing unit, the distance between the first effective pressure sensing unit and the target artery blood vessel, and the distance between the target secondary pressure sensing unit and the target artery blood vessel. For detailed content, refer to the foregoing related content. Details are not described herein again.

In some embodiments, the pressure data includes a plurality of static pressures and a dynamic pressure corresponding to each static pressure. In this case, an implementation process of correcting, based on the tissue attenuation coefficient corresponding to the at least one effective pressure sensing unit, the pressure data collected by the at least one effective pressure sensing unit includes: for any effective pressure sensing unit of the at least one effective pressure sensing unit, subtracting a tissue attenuation coefficient corresponding to the effective pressure sensing unit from pressure data of the effective pressure sensing unit separately in a plurality of absolute pressures to obtain a plurality of corrected static pressures, adding the tissue attenuation coefficient corresponding to the effective pressure sensing unit to the pressure data of the effective pressure sensing unit separately in a plurality of dynamic pressures to obtain a plurality of corrected dynamic pressures, and using the plurality of corrected static pressures and the plurality of corrected dynamic pressures as first pressure data of the effective pressure sensing unit, so as to implement correction of the pressure data collected by the effective pressure sensing unit. Each effective pressure sensing unit of the at least one effective pressure sensing unit is processed in a same manner, so as to implement correction of the pressure data collected by each effective pressure sensing unit of the at least one effective pressure sensing unit.

For a pressure (that is, a static pressure) applied by the air bladder, the pressure applied by the air bladder first reaches the absolute pressure sensing unit, and then reaches the target artery blood vessel after being attenuated by the human tissue. A static pressure experienced by the target artery blood vessel is less than a static pressure collected by the absolute pressure sensing unit. Therefore, the tissue attenuation coefficient corresponding to the effective pressure sensing unit can be separately subtracted from the plurality of static pressures, so that the static pressures can be corrected.

For a pulse wave generated by the target artery blood vessel, the pulse wave can reach the absolute pressure sensing unit only after being emitted from the target artery blood vessel and attenuated by the human tissue. A dynamic pressure generated by the target artery blood vessel is greater than a dynamic pressure collected by the absolute pressure sensing unit. Therefore, the tissue attenuation coefficient corresponding to the effective pressure sensing unit is separately added to the plurality of dynamic pressures, so that the dynamic pressures can be corrected.

**Step 1604:** Determine a blood pressure of the user based on the arterial pressure data corresponding to the at least one artery blood vessel.

When quantities of artery blood vessels are different, implementations of determining the blood pressure of the user based on the arterial pressure data corresponding to the at least one artery blood vessel are different. The following separately describes the implementations.

When a quantity of the at least one artery blood vessel is one, the wearable device may directly determine the blood pressure of the user based on the arterial pressure data corresponding to the artery blood vessel and according to a related algorithm.

When the quantity of the at least one artery blood vessel is at least two, that is, the at least one artery blood vessel includes at least two artery blood vessels, there are a plurality of implementations of determining the blood pressure of the user based on arterial pressure data corresponding to the at least two artery blood vessels. The following describes two implementations.

**In a first implementation,** the arterial pressure data includes static pressures respectively corresponding to a plurality of moments and a dynamic pressure corresponding to each static pressure, the static pressure represents a pressure applied by the air bladder, the dynamic pressure represents a pulsation status of a corresponding artery blood vessel under compression of the static pressure, and the at least two artery blood vessels are respectively corresponding to different weights. In this case, dynamic pressures corresponding to a same moment in the dynamic pressures of the arterial pressure data respectively corresponding to the at least two artery blood vessels are separately multiplied by respective corresponding weights, and then are added to obtain a plurality of superposed dynamic pressures, static pressures corresponding to a same moment in the dynamic pressures of the arterial pressure data respectively corresponding to the at least two artery blood vessels are separately multiplied by respective corresponding weights, and then are added to obtain a plurality of superposed static pressures, the plurality of superposed dynamic pressures and the plurality of superposed static pressures are used as the superposed arterial pressure data, and the blood pressure of the user is determined based on the superposed arterial pressure data and according to a related algorithm.

For example, if the quantity of artery blood vessels is 2, the two artery blood vessels are respectively an artery blood vessel 1 and an artery blood vessel 2, a weight corresponding to the artery blood vessel 1 is a weight 1, and a weight corresponding to the artery blood vessel 2 is a weight 2. Arterial pressure data respectively corresponding to the artery blood vessel 1 and the artery blood vessel 2 includes static pressures respectively corresponding to two moments and a dynamic pressure corresponding to each static pressure, and the two moments are a moment 1 and a moment 2 respectively. The moment 1 is used as an example. A static pressure corresponding to the artery blood vessel 1 at the moment 1 is a static pressure 11, a dynamic pressure corresponding to the moment 1 is a dynamic pressure 11, a static pressure corresponding to the artery blood vessel 2 at the moment 1 is a static pressure 21, and a dynamic pressure corresponding to the moment 1 is a dynamic pressure 21.

In this case, the static pressure 11 is multiplied by the weight 1, the static pressure 21 is multiplied by the weight 2, and the static pressure 11 obtained after the multiplication by the weight and the static pressure 12 obtained after the multiplication by the weight are added, to obtain a superposed static pressure corresponding to the moment 1. The dynamic pressure 11 is multiplied by the weight 1, the dynamic pressure 21 is multiplied by the weight 2, and the dynamic pressure 11 obtained after the multiplication by the weight and the dynamic pressure 12 obtained after the multiplication by the weight are added, to obtain a superposed dynamic pressure corresponding to the moment 1.

**In a second implementation,** the arterial pressure data includes a plurality of static pressures and a dynamic pressure corresponding to each static pressure, the static pressure represents a pressure applied by the air bladder, and the dynamic pressure represents a pulsation status of a corresponding artery blood vessel under compression of the static pressure. In this case, in some embodiments, the wearable device may superpose, based on the static pressure in the arterial pressure data respectively corresponding to the at least two artery blood vessels, the dynamic pressure in the arterial pressure data respectively corresponding to the at least two artery blood vessels, to obtain superposed arterial pressure data, and determine the blood pressure of the user based on the superposed arterial pressure data and according to a related algorithm.

Optionally, the at least two artery blood vessels are respectively corresponding to different weights. In this case, dynamic pressures corresponding to a same static pressure in the dynamic pressure data corresponding to the at least two artery blood vessels are respectively multiplied by respective weights, and then added to obtain a plurality of superposed dynamic pressures, and the plurality of superposed dynamic pressures and static pressures respectively corresponding to the plurality of superposed dynamic pressures are used as the superposed arterial pressure data.

It should be noted that a sum of weights respectively corresponding to the at least two artery blood vessels is 1.

For example, if the quantity of artery blood vessels is 2, the two artery blood vessels are respectively an artery blood vessel 1 and an artery blood vessel 2, a weight corresponding to the artery blood vessel 1 is a weight 1, and a weight corresponding to the artery blood vessel 2 is a weight 2. Arterial pressure data corresponding to the artery blood vessel 1 includes a static pressure 11 and a dynamic pressure 11 corresponding to the static pressure 11, and arterial pressure data corresponding to the artery blood vessel 2 includes a static pressure 21 and a dynamic pressure 21 corresponding to the static pressure 21.

If the static pressure 11 is equal to the static pressure 21, the dynamic pressure 11 is multiplied by the weight 1, the dynamic pressure 21 is multiplied by the weight 2, and the dynamic pressure 11 obtained after the multiplication by the weight and the dynamic pressure 12 obtained after the multiplication by the weight are added, to obtain a superposed dynamic pressure corresponding to the static pressure 11 (or the static pressure 21).

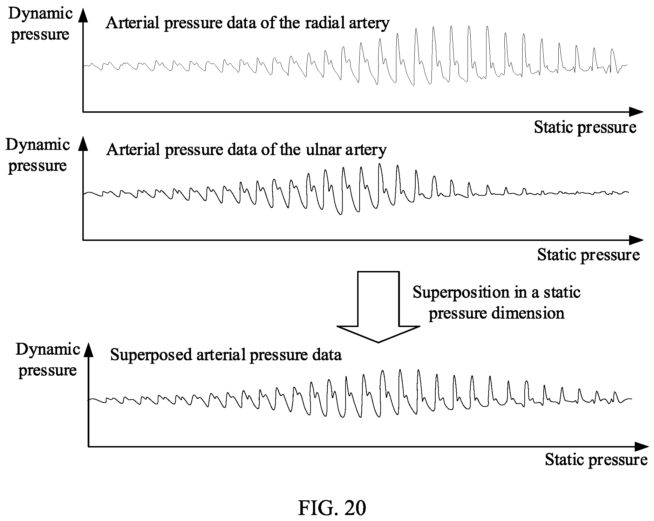

When the at least two artery blood vessels are an ulnar artery and a radial artery, based on the foregoing description, due to different depths of the ulnar artery and the radial artery in the human tissue, irregular bones of the wrist of the human body, and impact of a wearing manner of the user, a pulse wave signal in the air bladder obtained by superimposing pulse pulsation of the ulnar artery and the radial artery in time domain cannot accurately represent a pulsation status of the artery blood vessel of the user in a pressurization process, and has a large error. Consequently, accuracy of a blood pressure result is poor. Refer to FIG. 20. In this embodiment of this application, the arterial pressure data respectively corresponding to the at least two artery blood vessels can be superposed in a static pressure dimension, thereby fundamentally avoiding a problem that a pulse wave signal error is large due to simple superposition in time domain, and further improving accuracy of blood pressure measurement.

Because the sensor array includes a plurality of absolute pressure sensing units, and a pressure measured by the absolute pressure sensing unit is relative to a vacuum pressure, the pressure measured by the absolute pressure sensing units is not affected by a change of an atmospheric pressure and can accurately reflect a real pressure status. Because the at least one sensor array is on the side of the air bladder away from the wearable strap, in this case, when the user wears the wearable device, the sensor array can be in contact with skin of the user, and collect a pressure data at the contact position. The pressure data is relative to the vacuum pressure. Compared with a method for collecting pressure data inside the air bladder, the pressure data collected in this embodiment of this application can accurately represent a pressure actually experienced by a corresponding contact position. Therefore, accuracy of a finally determined blood pressure of the user is ensured. In addition, in this embodiment of this application, considering that a size of an artery blood vessel is smaller relative to a size of a sensor array, in this embodiment of this application, an absolute pressure sensing unit (also referred to as an effective pressure sensing unit) that can effectively represent an artery blood vessel pulsation status of a user is determined from a plurality of absolute pressure sensing units included in the sensor array, and then arterial pressure data respectively corresponding to the artery blood vessel is determined based on pressure data collected by the effective pressure sensing unit. In this way, accuracy of finally determined arterial pressure data is ensured. In addition, because the arterial pressure data can represent a pressure actually experienced by a corresponding artery blood vessel in a blood pressure measurement process, accuracy of a blood pressure result determined based on the arterial pressure data corresponding to the artery blood vessel is high. In addition, when arterial pressure data corresponding to at least two artery blood vessels is determined, in this embodiment of this application, the blood pressure of the user can be further determined based on the arterial pressure data corresponding to the at least two artery blood vessels, thereby further improving accuracy of blood pressure measurement.

When absolute pressure sensing units in two adjacent rows of the plurality of absolute pressure sensing units included in the sensor array are staggered, it can be effectively ensured that the sensor array can collect a pulse wave signal with optimal signal strength, thereby ensuring accuracy of finally determined blood pressure of the user. In this embodiment of this application, considering attenuation of the pulse wave of the target artery blood vessel by the human tissue, the pressure data collected by the effective pressure sensing unit is corrected by calculating the tissue attenuation coefficient, so that the corrected pressure data collected by the effective pressure sensing unit can accurately reflect a pulse of the artery blood vessel, thereby ensuring accuracy and reliability of finally determined blood pressure of the user.

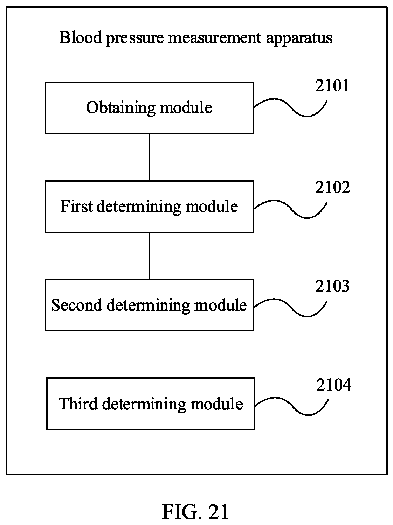

FIG. 21 is a diagram of a structure of a blood pressure measurement apparatus according to an embodiment of this application. The blood pressure measurement apparatus may be implemented as a part or all of the foregoing wearable device by using software, hardware, or a combination thereof. The apparatus includes an obtaining module 2101, a first determining module 2102, a second determining module 2103, and a third determining module 2104.

The obtaining module 2101 is configured to obtain a pressure data set separately collected by at least one sensor array, where the pressure data set is collected by a plurality of absolute pressure sensing units included in a corresponding sensor array in a process of inflating and pressurizing an air bladder or deflating and depressurizing the air bladder. For a detailed implementation process, refer to corresponding content in the foregoing embodiments. Details are not described herein again.

The first determining module 2102 is configured to determine, based on the pressure data set collected by the at least one sensor array, an effective pressure sensing unit from absolute pressure sensing units included in the at least one sensor array, where pressure data collected by the effective pressure sensing unit is able to effectively represent a pulsation status of an artery blood vessel of a user. For a detailed implementation process, refer to corresponding content in the foregoing embodiments. Details are not described herein again.

The second determining module 2103 is configured to determine, based on the pressure data collected by the effective pressure sensing unit in the at least one sensor array, arterial pressure data respectively corresponding to the at least one artery blood vessel, where the arterial pressure data represents a pressure actually experienced by a corresponding artery blood vessel in a blood pressure measurement process. For a detailed implementation process, refer to corresponding content in the foregoing embodiments. Details are not described herein again.

The third determining module 2104 is configured to determine a blood pressure of the user based on the arterial pressure data corresponding to the at least one artery blood vessel. For a detailed implementation process, refer to corresponding content in the foregoing embodiments. Details are not described herein again.

Optionally, the pressure data set includes a plurality of groups of pressure data collected by a plurality of absolute pressure sensing units, and each group of pressure data includes pressure data at a plurality of moments.

The first determining module 2102 is specifically configured to:
determine, based on a first pressure data set, a plurality of groups of candidate pressure sensing units from absolute pressure sensing units included in a first sensor array;
where the first pressure data set is a pressure data set collected by the first sensor array, the first sensor array is any one of the at least one sensor array, the plurality of groups of candidate pressure sensing units are in a one-to-one correspondence with a plurality of first moments, positions of a same group of candidate pressure sensing units are consecutive, and a difference between pressure data collected by the same group of candidate pressure sensing units at a corresponding first moment falls within a pressure fluctuation range, where the first moment is one of the plurality of moments, and the first artery blood vessel is an artery blood vessel corresponding to the first sensor array; and
determine an effective pressure sensing unit in the first sensor array from the plurality of groups of candidate pressure sensing units.

Optionally, the apparatus further includes:
a fourth determining module, configured to determine a tissue attenuation coefficient corresponding to at least one effective pressure sensing unit in a target sensor array, where the tissue attenuation coefficient indicates an attenuation status of a pulse wave of a target artery blood vessel caused by a human tissue, the target artery blood vessel is an artery blood vessel corresponding to the target sensor array, and the target sensor array is any sensor array in the at least one sensor array; and
a correction module, configured to correct, based on the tissue attenuation coefficient corresponding to the at least one effective pressure sensing unit, pressure data collected by the at least one effective pressure sensing unit.

Optionally, the fourth determining module is specifically configured to:
determine a distance between the at least one effective pressure sensing unit and the target artery blood vessel;
determine a unit attenuation coefficient, where the unit attenuation coefficient is an attenuation status of the pulse wave of the target artery blood vessel caused by a human tissue with a unit thickness; and
determine, based on the unit attenuation coefficient and the distance between the at least one effective pressure sensing unit and the target artery blood vessel, the tissue attenuation coefficient respectively corresponding to the at least one effective pressure sensing unit.

Optionally, the arterial pressure data includes a plurality of static pressures and a dynamic pressure corresponding to each static pressure, the at least one artery blood vessel includes at least two artery blood vessels, the static pressure represents a pressure applied by the air bladder, and the dynamic pressure represents a pulsation status of a corresponding artery blood vessel under compression of the static pressure.

The third determining module 2104 is specifically configured to:
superpose, based on static pressures in arterial pressure data respectively corresponding to the at least two artery blood vessels, dynamic pressures in the arterial pressure data respectively corresponding to the at least two artery blood vessels, to obtain superposed arterial pressure data; and
determine the blood pressure of the user based on the superposed arterial pressure data.

In this embodiment of this application, because the sensor array includes a plurality of absolute pressure sensing units, and a pressure measured by the absolute pressure sensing unit is relative to a vacuum pressure, the pressure measured by the absolute pressure sensing units is not affected by a change of an atmospheric pressure and can accurately reflect a real pressure status. Because the at least one sensor array is on the side of the air bladder away from the wearable strap, in this case, when the user wears the wearable device, the sensor array can be in contact with skin of the user, and collect a pressure data at the contact position. The pressure data is relative to the vacuum pressure. Compared with a method for collecting pressure data inside the air bladder, the pressure data collected in this embodiment of this application can accurately represent a pressure actually experienced by a corresponding contact position. Therefore, accuracy of a finally determined blood pressure of the user is ensured. In addition, in this embodiment of this application, considering that a size of an artery blood vessel is smaller relative to a size of a sensor array, in this embodiment of this application, an absolute pressure sensing unit (also referred to as an effective pressure sensing unit) that can effectively represent an artery blood vessel pulsation status of a user is determined from a plurality of absolute pressure sensing units included in the sensor array, and then arterial pressure data respectively corresponding to the artery blood vessel is determined based on pressure data collected by the effective pressure sensing unit. In this way, accuracy of finally determined arterial pressure data is ensured. In addition, because the arterial pressure data can represent a pressure actually experienced by a corresponding artery blood vessel in a blood pressure measurement process, accuracy of a blood pressure result determined based on the arterial pressure data corresponding to the artery blood vessel is high. In addition, when arterial pressure data corresponding to at least two artery blood vessels is determined, in this embodiment of this application, the blood pressure of the user can be further determined based on the arterial pressure data corresponding to the at least two artery blood vessels, thereby further improving accuracy of blood pressure measurement.

It should be noted that, when the blood pressure measurement apparatus provided in the foregoing embodiment performs blood pressure measurement, division into the foregoing functional modules is merely described by using an example. In actual application, the foregoing functions may be allocated to different functional modules for implementation based on a requirement. In other words, an internal structure of the apparatus is divided into different functional modules, to implement all or some of the functions described above. In addition, the blood pressure measurement apparatus provided in the foregoing embodiment is based on the same concept as the blood pressure measurement method embodiment. For a specific implementation process thereof, refer to the method embodiment. Details are not described herein again.

An embodiment of this application further provides a computer-readable storage medium. The storage medium stores instructions. When the instructions are run on a computer, the computer is enabled to perform steps of the blood pressure measurement method in the foregoing embodiment, or perform steps of the blood pressure measurement method in the foregoing embodiment.

An embodiment of this application further provides a computer program product including instructions. When the instructions are run on a computer, the computer is enabled to perform steps of the blood pressure measurement method in the foregoing embodiment. In other words, a computer program is provided. When the computer program is run on a computer, the computer is enabled to perform steps of the blood pressure measurement method in the foregoing embodiment.

All or some of the foregoing embodiments may be implemented by software, hardware, firmware, or any combination thereof. When software is used to implement embodiments, all or a part of embodiments may be implemented in a form of a computer program product. The computer program product includes one or more computer instructions. When the computer instructions are loaded and executed on the computer, the procedure or functions according to embodiments of this application are all or partially generated. The computer may be a general-purpose computer, a dedicated computer, a computer network, or other programmable apparatuses. The computer instructions may be stored in a computer-readable storage medium, or may be transmitted from a computer-readable storage medium to another computer-readable storage medium. For example, the computer instructions may be transmitted from a website, a computer, a server or a data center to another website, computer, server or data center in a wired (for example, a coaxial cable, an optical fiber, or a digital subscriber line (digital subscriber line, DSL)) or wireless (for example, infrared, radio, or microwave) manner. The computer-readable storage medium may be any usable medium accessible by the computer, or a data storage device, such as a server or a data center, integrating one or more usable media. The usable medium may be a magnetic medium (for example, a floppy disk, a hard disk, or a magnetic tape), an optical medium (for example, a digital versatile disc (digital versatile disc, DVD)), a semiconductor medium (for example, a solid-state disk (solid-state disk, SSD)), or the like. It should be noted that the computer-readable storage medium mentioned in embodiments of this application may be a non-volatile storage medium, that is, may be a non-transitory storage medium.

It should be understood that "a plurality of" in this specification means two or more. In descriptions of embodiments of this application, "/" indicates "or" unless otherwise specified. For example, A/B may indicate A or B. In this specification, "and/or" describes only an association relationship between associated objects and indicates that three relationships may exist. For example, A and/or B may indicate the following three cases: Only A exists, both A and B exist, and only B exists. In addition, to clearly describe technical solutions in embodiments of this application, terms such as "first" and "second" are used in embodiments of this application to distinguish between same items or similar items that provide basically same functions or purposes. A person skilled in the art may understand that the terms such as "first" and "second" do not limit a quantity or an execution sequence, and the terms such as "first" and "second" do not indicate a definite difference.

It should be noted that information (including but not limited to user equipment information, personal information of a user, and the like), data (including but not limited to data used for analysis, stored data, displayed data, and the like), and signals in embodiments of this application are used under authorization by the user or full authorization by all parties, and capturing, use, and processing of related data need to conform to related laws, regulations, and standards of related countries and regions. For example, the pressure data set separately collected by the at least one sensor array in embodiments of this application is obtained with sufficient authorization.

The foregoing descriptions are merely embodiments of this application, but are not intended to limit this application. Any modification, equivalent replacement, or improvement made without departing from the spirit and principle of this application should fall within the protection scope of this application.

## Claims

1. A wearable device, wherein the wearable device comprises a wearable strap, an inflatable component, at least one sensor array, and a processor, and the inflatable component comprises an air pump and an air bladder;
the air bladder is on an inner side of the wearable strap, the air bladder is distributed along a length direction of the wearable strap, and the air pump is configured to inflate and pressurize the air bladder and then deflate and depressurize the air bladder in a process in which a user measures a blood pressure;
the at least one sensor array is on a side of the air bladder away from the wearable strap, and when the user wears the wearable device, the at least one sensor array corresponds to a position of at least one artery blood vessel of the user;
the sensor array comprises a plurality of absolute pressure sensing units, a size of the absolute pressure sensing unit in a first direction is not greater than a diameter of a corresponding artery blood vessel, and the first direction is perpendicular to a flow direction of the corresponding artery blood vessel; and
the sensor array is configured to collect a pressure experienced by a corresponding artery blood vessel in the process in which the user measures the blood pressure, and the processor is configured to determine the blood pressure of the user based on a pressure data set collected by the at least one sensor array.

2. The wearable device according to claim 1, wherein a column direction of the sensor array is the same as a flow direction of the corresponding artery blood vessel, the plurality of absolute pressure sensing units are arranged in a manner of M rows and N columns, a spacing between two adjacent absolute pressure sensing units in a same row is not greater than a diameter of the corresponding artery blood vessel, M is an integer greater than or equal to 1, and N is an integer greater than 1.

3. The wearable device according to claim 2, wherein absolute pressure sensing units in two adjacent rows of the plurality of absolute pressure sensing units are staggered.

4. The wearable device according to claim 1, wherein the at least one sensor array comprises a first array and/or a second array, the first array corresponds to a radial artery blood vessel, and the second array corresponds to an ulnar artery blood vessel.

5. The wearable device according to claim 1, wherein a size of the sensor array in the first direction is greater than three times a diameter of the corresponding artery blood vessel.

6. A blood pressure measurement method applied to the wearable device according to any one of claims 1 to 5, wherein the method comprises:
obtaining a pressure data set separately collected by the at least one sensor array, wherein the pressure data set is collected by a plurality of absolute pressure sensing units comprised in a corresponding sensor array in a process of inflating and pressurizing the air bladder or deflating and depressurizing the air bladder;
determining, based on the pressure data set collected by the at least one sensor array, an effective pressure sensing unit from absolute pressure sensing units comprised in the at least one sensor array, wherein pressure data collected by the effective pressure sensing unit is able to effectively represent a pulsation status of an artery blood vessel of the user;
determining, based on the pressure data collected by the effective pressure sensing unit in the at least one sensor array, arterial pressure data respectively corresponding to the at least one artery blood vessel, wherein the arterial pressure data represents a pressure actually experienced by a corresponding artery blood vessel in a blood pressure measurement process; and
determining a blood pressure of the user based on the arterial pressure data corresponding to the at least one artery blood vessel.

7. The method according to claim 6, wherein the pressure data set comprises a plurality of groups of pressure data collected by the plurality of absolute pressure sensing units, and each group of pressure data comprises pressure data at a plurality of moments; and
determining, based on the pressure data set collected by the at least one sensor array, the effective pressure sensing unit from the absolute pressure sensing units comprised in the at least one sensor array comprises:
determining, based on a first pressure data set, a plurality of groups of candidate pressure sensing units from absolute pressure sensing units comprised in a first sensor array;
wherein the first pressure data set is a pressure data set collected by the first sensor array, the first sensor array is any one of the at least one sensor array, the plurality of groups of candidate pressure sensing units are in a one-to-one correspondence with a plurality of first moments, positions of a same group of candidate pressure sensing units are consecutive, and a difference between pressure data collected by the same group of candidate pressure sensing units at a corresponding first moment falls within a pressure fluctuation range, wherein the first moment is one of the plurality of moments, and the first artery blood vessel is an artery blood vessel corresponding to the first sensor array; and
determining an effective pressure sensing unit in the first sensor array from the plurality of groups of candidate pressure sensing units.

8. The method according to claim 6, wherein before determining, based on the pressure data collected by the effective pressure sensing unit in the at least one sensor array, the arterial pressure data respectively corresponding to the at least one artery blood vessel, the method further comprises:
determining a tissue attenuation coefficient corresponding to at least one effective pressure sensing unit in a target sensor array, wherein the tissue attenuation coefficient indicates an attenuation status of a pulse wave of a target artery blood vessel caused by a human tissue, the target artery blood vessel is an artery blood vessel corresponding to the target sensor array, and the target sensor array is any sensor array in the at least one sensor array; and
correcting, based on the tissue attenuation coefficient corresponding to the at least one effective pressure sensing unit, pressure data collected by the at least one effective pressure sensing unit.

9. The method according to claim 8, wherein determining the tissue attenuation coefficient corresponding to the at least one effective pressure sensing unit in the target sensor array comprises:
determining a distance between the at least one effective pressure sensing unit and the target artery blood vessel;
determining a unit attenuation coefficient, wherein the unit attenuation coefficient is an attenuation status of the pulse wave of the target artery blood vessel caused by a human tissue with a unit thickness; and
determining, based on the unit attenuation coefficient and the distance between the at least one effective pressure sensing unit and the target artery blood vessel, the tissue attenuation coefficient respectively corresponding to the at least one effective pressure sensing unit.

10. The method according to claim 6, wherein the arterial pressure data comprises a plurality of static pressures and a dynamic pressure corresponding to each static pressure, the at least one artery blood vessel comprises at least two artery blood vessels, the static pressure represents a pressure applied by the air bladder, and the dynamic pressure represents a pulsation status of a corresponding artery blood vessel under compression of the static pressure; and
determining the blood pressure of the user based on the arterial pressure data respectively corresponding to the at least one artery blood vessel comprises:
superposing, based on static pressures in arterial pressure data respectively corresponding to the at least two artery blood vessels, dynamic pressures in the arterial pressure data respectively corresponding to the at least two artery blood vessels, to obtain superposed arterial pressure data; and
determining the blood pressure of the user based on the superposed arterial pressure data.

11. A blood pressure measurement apparatus, comprised in the wearable device according to any one of claims 1 to 5, wherein the apparatus comprises:
an obtaining module, configured to obtain a pressure data set separately collected by the at least one sensor array, wherein the pressure data set is collected by a plurality of absolute pressure sensing units comprised in a corresponding sensor array in a process of inflating and pressurizing the air bladder or deflating and depressurizing the air bladder;
a first determining module, configured to determine, based on the pressure data set collected by the at least one sensor array, an effective pressure sensing unit from absolute pressure sensing units comprised in the at least one sensor array, wherein pressure data collected by the effective pressure sensing unit is able to effectively represent a pulsation status of an artery blood vessel of the user;
a second determining module, configured to determine, based on the pressure data collected by the effective pressure sensing unit in the at least one sensor array, arterial pressure data respectively corresponding to the at least one artery blood vessel, wherein the arterial pressure data represents a pressure actually experienced by a corresponding artery blood vessel in a blood pressure measurement process; and
a third determining module, configured to determine a blood pressure of the user based on the arterial pressure data corresponding to the at least one artery blood vessel.

12. The apparatus according to claim 11, wherein the pressure data set comprises a plurality of groups of pressure data collected by the plurality of absolute pressure sensing units, and each group of pressure data comprises pressure data at a plurality of moments; and
the first determining module is specifically configured to:
determine, based on a first pressure data set, a plurality of groups of candidate pressure sensing units from absolute pressure sensing units comprised in a first sensor array;
wherein the first pressure data set is a pressure data set collected by the first sensor array, the first sensor array is any one of the at least one sensor array, the plurality of groups of candidate pressure sensing units are in a one-to-one correspondence with a plurality of first moments, positions of a same group of candidate pressure sensing units are consecutive, and a difference between pressure data collected by the same group of candidate pressure sensing units at a corresponding first moment falls within a pressure fluctuation range, wherein the first moment is one of the plurality of moments, and the first artery blood vessel is an artery blood vessel corresponding to the first sensor array; and
determine an effective pressure sensing unit in the first sensor array from the plurality of groups of candidate pressure sensing units.

13. The apparatus according to claim 11, wherein the apparatus further comprises:
a fourth determining module, configured to determine a tissue attenuation coefficient corresponding to at least one effective pressure sensing unit in a target sensor array, wherein the tissue attenuation coefficient indicates an attenuation status of a pulse wave of a target artery blood vessel caused by a human tissue, the target artery blood vessel is an artery blood vessel corresponding to the target sensor array, and the target sensor array is any sensor array in the at least one sensor array; and
a correction module, configured to correct, based on the tissue attenuation coefficient corresponding to the at least one effective pressure sensing unit, pressure data collected by the at least one effective pressure sensing unit.

14. The apparatus according to claim 13, wherein the fourth determining module is specifically configured to:
determine a distance between the at least one effective pressure sensing unit and the target artery blood vessel;
determine a unit attenuation coefficient, wherein the unit attenuation coefficient is an attenuation status of the pulse wave of the target artery blood vessel caused by a human tissue with a unit thickness; and
determine, based on the unit attenuation coefficient and the distance between the at least one effective pressure sensing unit and the target artery blood vessel, the tissue attenuation coefficient respectively corresponding to the at least one effective pressure sensing unit.

15. The apparatus according to claim 11, wherein the arterial pressure data comprises a plurality of static pressures and a dynamic pressure corresponding to each static pressure, the at least one artery blood vessel comprises at least two artery blood vessels, the static pressure represents a pressure applied by the air bladder, and the dynamic pressure represents a pulsation status of a corresponding artery blood vessel under compression of the static pressure; and
the third determining module is specifically configured to:
superpose, based on static pressures in arterial pressure data respectively corresponding to the at least two artery blood vessels, dynamic pressures in the arterial pressure data respectively corresponding to the at least two artery blood vessels, to obtain superposed arterial pressure data; and
determine the blood pressure of the user based on the superposed arterial pressure data.

16. A computer-readable storage medium, wherein the storage medium stores instructions, and when the instructions are run on the computer, the computer is enabled to perform steps of the method according to any one of claims 6 to 10.

17. A computer program, wherein the computer program comprises instructions, and when the instructions are run on the computer, the computer is enabled to perform steps of the method according to any one of claims 6 to 10.
